# EUROPEAN PATENT SPECIFICATION

(11) **EP 4 366 805 B1**
(45) Date of publication and mention of the grant of the patent: **17.12.2025**
(21) Application number: 22777312.4
(22) Date of filing: 25.08.2022
(51) Int. Cl.: A61M 15/00, A61M 16/01, A61M 16/18, A61M 15/06

(54) **INHALATION DEVICE AND INHALATION SYSTEM**
INHALATIONSVORRICHTUNG UND INHALATIONSSYSTEM
DISPOSITIF D'INHALATION ET SYSTÈME D'INHALATION

(30) Priority: 25.08.2021 UA 202104788; 26.08.2021 UA 202104820 U; 26.08.2021 UA 202104821 U
(43) Date of publication of application: 15.05.2024
(73) Proprietor: M.T.K. Medical Center Limited Liability Company, Kyiv 03680 (UA)
(72) Inventor: GUMENIUK, Mykola, Kyiv, 03110 (UA)
(74) Representative: Lichtnecker, Markus Christoph
(86) International application number: PCT/IB2022/057982
(87) International publication number: WO 2023/026237

(56) References cited:
- JP-A- H01 124 451
- US-A1- 2018 207 390
- US-A1- 2021 001 060

## Description

### FIELD OF THE INVENTION

The disclosure relates to medical devices, namely to devices for inhalation of volatile anesthetics.

### BACKGROUND OF THE INVENTION

The use of volatile liquids as active substances or volatile liquids containing active substances is well known. One such example is halogen-containing volatile liquids. Halogen-containing volatile liquids are described as useful for inducing and/or maintaining anesthesia (including amnesia, muscle paralysis and/or sedation) and/or analgesia, and, therefore, they may be useful as volatile anesthetics and/or volatile analgesics.

The easiest way for inducing anesthesia using volatile anesthetics is inhalation, that is, inhaling a vapor of the volatile anesthetics by the patients.

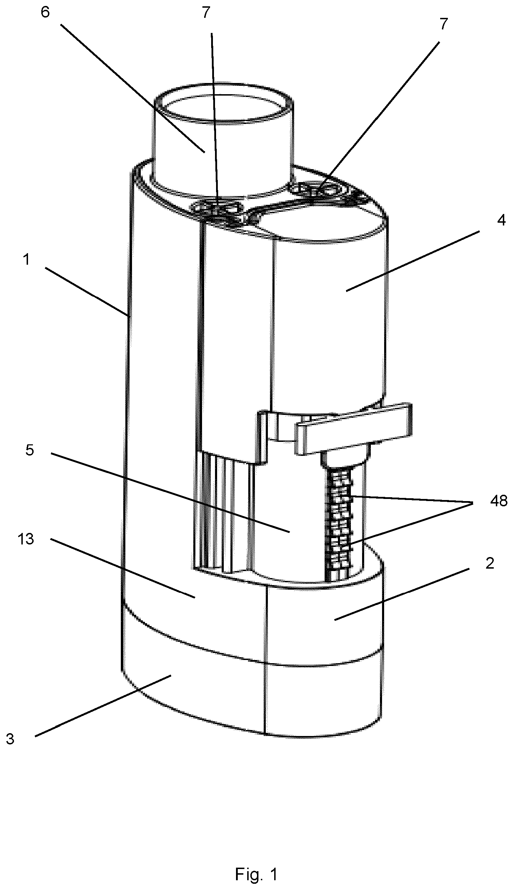

Russian patent for invention RU 2720169 C2 published on 24.04.2020 describes an inhalation device called "Green Whistle" and shown in Figure 1, that contains an evaporation chamber and a filter (the filter is also referred to as "AC chamber"). The "Green Whistle" inhalation device is intended for inhalation of the volatile anesthetic. such as methoxyflurane (Penthrox drug). The evaporation chamber has a cylindrical case, and the interior volume of the case is filled with fibers of polymer material. The case of the evaporation chamber has an open inlet orifice, into which methoxyflurane is poured and through which air flows, and an outlet orifice, through which a human inhales a mixture of the methoxyflurane vapor and air. The filter is intended to absorb the methoxyflurane vapor from the gas exhaled by a human, and it comprises activated carbon, and connects to the evaporation chamber.

The web page at https://www.arkhealth.com.au/354221/Penthrox-Combo-Pack-with-AC-Chamber-%28SCHEDULE-4%29/pd.php describes an inhalation system called "Penthrox Combo Pack with AC Chamber", which comprises "Green Whistle" inhalation device above and the Penthrox drug in the form of a bottle with methoxyflurane.

The "Green Whistle" inhalation device and "Penthrox Combo Pack with AC Chamber" inhalation system are used as follows. A human opens the bottle with methoxyflurane, pours a portion of methoxyflurane into the evaporation chamber through the inlet orifice in the evaporation chamber case, then attaches the filter to the evaporation chamber, brings the device to the mouth so that the outlet orifice of the evaporation chamber case is positioned between the human lips, and alternately inhales and exhales. US2021/001060 A1 relates to an inhaler device for inhalable liquids, in particular for the storage and/or administration of inhalable volatile liquids such as halogenated volatile liquids, to a patient.

A disadvantage of the known inhalation device and the known inhalation system is that, both during preparation for use of the device and after use of the device, vapors of the volatile anesthetic (methoxyflurane) are released into the air of the room where a patient is, and these vapors are inhaled by other people. This is related both to the design of the inhalation device itself, namely, the design of the evaporation chamber, and to the supply of the volatile anesthetic to the evaporation chamber, which is carried out using the usual pouring of the volatile anesthetic from the bottle to the evaporation chamber.

Another disadvantage of the known inhalation device is the impossibility of adjusting the dose of the volatile anesthetic inhaled by a patient. After pouring the volatile anesthetic into the evaporation chamber, the mixture of the volatile anesthetic vapor and air with a very high concentration of the volatile anesthetic in this mixture is immediately formed in the evaporation chamber. A patient suffering from pain tries to relieve pain as soon as possible, and, therefore, at the start of the inhalation, they instinctively inhale the mixture of the volatile anesthetic vapor and air quickly. The specific feature of the halogen-containing volatile anesthetics is that when they enter the human body in excess of a specific dose, it is possible that, instead of anesthesia (that is, a condition when a human is conscious, but at the same time the pain subsides), narcosis occurs, when a human loses consciousness. Therefore, the best option for inhalation with the volatile anesthetic is when a patient inhales small doses of the volatile anesthetic over a specific extended period of time.

The first objective technical problem to be solved is to improve the inhalation device with the aim of developing a device, that prevent the leakage of the volatile anesthetic vapor into the environment and enables adjusting the dose of the volatile anesthetic inhaled by a human, as well as expanding a range of means for the inhalation with the volatile anesthetics, preferably, means for the inhalation with methoxyflurane.

The second objective technical problem to be solved is the improvement of the inhalation system with the aim of developing a system, using of which does not cause the leakage of the volatile anesthetic vapor into the environment, and which enables adjusting the dose of the volatile anesthetic inhaled by a human, as well as expanding a range of means for the inhalation with the volatile anesthetics, preferably, means for the inhalation with methoxyflurane.

### SUMMARY OF THE INVENTION

The invention is defined by the appended claims. The present invention defines an inhalation device (1) comprising an evaporation chamber (2) and a filter (3) being connected to each other; wherein the filter (3) comprises a case (32) with inlet (33) and at least one outlet (34), in which the filter material (35) is located; wherein the evaporation chamber (2) comprises a hollow case (4) having walls isolating the interior space from the environment, a receiving element for the syringe (5) is configured to be installed with a syringe barrel filled with a volatile anesthetic, a connecting branch (6) connected to the wall of the hollow case (4), an exhalation channel (10) being located inside the hollow case (4) and dividing the interior space of the hollow case (4) into two spaces isolated from each other, such as the evaporation space, in which the evaporation of the volatile anesthetic and the formation of a mixture of the volatile anesthetic vapor and air occur, and the space in the exhalation channel (10) being intended for the flow of the gas exhaled by a human to the filter (3); at least one air valve (7) being configured to pass a portion of the air from the environment into the evaporation space of the hollow case (4) when a human inhales, and being positioned in the hollow case (4) so that the inlet end of the air valve (7) is connected to the walls of the hollow case (4) and the inlet orifice (19) of the air valve (7) is an orifice in the wall of the hollow case (4); an inhalation valve (8) being configured to pass a portion of the mixture of the volatile anesthetic vapor and air from the evaporation space of the hollow case (4) to the interior space of the connecting branch (6), when a human inhales, and being positioned in the hollow case (4) so that the outlet end of the inhalation valve (8) is connected to the wall of the hollow case (4), and the outlet orifice (24) of the inhalation valve (8) is an orifice in the wall of the hollow case (4); the exhalation valve (9) being configured to remove a portion of the gas exhaled by a human from the interior space of the connecting branch (6) into the space of the exhalation channel (10), when a human exhales, and being positioned in the hollow case (4) so that the inlet end of the exhalation valve (9) is connected to the wall of the hollow case (4), and the inlet orifice (27) of the exhalation valve (9) is an orifice in the wall of the hollow case (4); and a filler (11) being located in the evaporation space of the hollow case (4); wherein the receiving element for the syringe (5) is configured in the form of a tube (12) located on the side wall (13) of the hollow case (4) and is configured so that the first end of the tube (12) has an orifice for the syringe barrel (14), the second end of the tube (12) has an end wall (15) with an inlet channel (16) being intended for connecting a tip of the syringe barrel to the evaporation chamber (2) and the passage of the volatile anesthetic from the syringe barrel to the evaporation space of the hollow case (4); the connecting branch (6) is configured in the form of a pipe having a rounded or oval shape in the cross-section; wherein the outlet end with an outlet orifice (28) of the exhalation valve (9) is connected to the inlet end of the exhalation channel (10), an outlet end (31) of the exhalation channel (10) is connected to the inlet (33) of the filter (3); wherein the connecting branch (6), inhalation valve (8), and exhalation valve (9) are mutually spatially arranged so that the inhalation valve (8) and the exhalation valve (9) are positioned next to each other at a short distance from each other; and wherein the mixture of the volatile anesthetic vapor and air exits the outlet orifice (24) of the inhalation valve (8) and passes to the interior space of the connecting branch (6); and the gas exhaled by a human from the interior space of the connecting branch (6) enters the inlet (27) of the exhalation valve (9).

In one aspect, the inhalation device (1) can comprise a mouthpiece to be mounted on the connecting branch (6) or inserted into the connecting branch (6).

In one aspect, a portion of the side wall (13) of the hollow case (4), on which the receiving element for the syringe (5) is positioned, can be configured in the form of a platform with a flat surface (36) being intended for the contacting with a lateral side of a syringe barrel flange with a flat surface.

In one aspect, the side wall (13) of the hollow case (4), on which the receiving element for the syringe (5) is positioned, can comprise a transverse groove with a rounded surface (37) being intended for inserting the lateral side of the syringe barrel flange with the rounded surface in it.

In one aspect, the inhalation device (1) can comprise a button (41) being connected to the side wall (13) of the hollow case (4) and being able to move relatively to the side wall (13) of the hollow case (4), and being configured to drive a syringe piston; wherein the button (41) is configured to comprise at least one longitudinal connecting ledge (42) and at least one connecting groove (43) on the lateral side, and wherein the side wall (13) of the hollow case (4) comprises at least one longitudinal connecting groove (44) and at least one longitudinal connecting ledge (45); wherein the button (41) is connected to the side wall (13) of the hollow case (4) using the longitudinal connecting ledge (42), the connecting groove (43), the longitudinal connecting groove (44) and the longitudinal connecting ledge (45) so that the longitudinal connecting ledge (42) is spatially positioned in the longitudinal connecting groove (44), and the longitudinal connecting ledge (45) is spatially positioned in the connecting groove (43).

In one aspect, the button (41) can comprise at least one locking element (46), wherein each locking element (46) comprises a contact ledge (47); wherein a tube (12) of the receiving element for the syringe (5) comprises at least one series of the locking ledges (48) on the outer surface of the tube (12) of the receiving element for the syringe (5); wherein the locking element (46) and the series of the locking ledges (48) are configured to enable the contact ledge (47) to contact with the locking ledges (48), when the button (41) moves relatively to the side wall (13) of the hollow case (4).

In one aspect, the contact ledge (47) and the locking ledges (48) can be configured to enable the move of the button (41) relatively to the side wall (13) of the hollow case (4) in one direction only.

In one aspect, the button (41) can comprise a rod (49).

In one aspect, the rod (49) can have a cross-shaped cross-section.

In one aspect, the evaporation chamber (2) and the filter (3) can have a detachable connection with each other.

In one aspect, the volatile anesthetic is methoxyflurane.

The present disclosure also relates to an inhalation system comprising an inhalation device (1) and a syringe filled with the volatile anesthetic; wherein an inhalation device (1) comprises an evaporation chamber (2) and a filter (3) being connected to each other; wherein the filter (3) is configured to comprise a case (32) with inlet (33) and at least one outlet (34), in which the filter material (35) being located; wherein the evaporation chamber (2) is configured to comprise a hollow case (4) having walls isolating the interior space from the environment, a receiving element for the syringe (5) is configured to be installed with a syringe barrel filled with a volatile anesthetic, a connecting branch (6) being connected to the wall of the hollow case (4), an exhalation channel (10) being located inside the hollow case (4) and dividing the interior space of the hollow case (4) into two spaces isolated from each other, such as the evaporation space, in which the evaporation of the volatile anesthetic and the formation of a mixture of the volatile anesthetic vapor and air occur, and the space in the exhalation channel (10) being intended for the flow of the gas exhaled by a human to the filter (3); at least one air valve (7) is configured to pass a portion of the air from the environment into the evaporation space of the hollow case (4) when a human inhales, and being positioned in the hollow case (4) so that the inlet end of the air valve (7) being connected to the walls of the hollow case (4) and the inlet orifice (19) of the air valve (7) being an orifice in the wall of the hollow case (4); an inhalation valve (8) being configured to pass a portion of the mixture of the volatile anesthetic vapor and air from the evaporation space of the hollow case (4) to the interior space of the connecting branch (6), when a human inhales, and being positioned in the hollow case (4) so that the outlet end of the inhalation valve (8) is connected to the wall of the hollow case (4), and the outlet orifice (24) of the inhalation valve (8) is an orifice in the wall of the hollow case (4); the exhalation valve (9) being configured to remove a portion of the gas exhaled by a human from the interior space of the connecting branch (6) into the space of the exhalation channel (10), when a human exhales, and being positioned in the hollow case (4) so that the inlet end of the exhalation valve (9) is connected to the wall of the hollow case (4), and the inlet orifice (27) of the exhalation valve (9) is an orifice in the wall of the hollow case (4); and a filler (11) being located in the evaporation space of the hollow case (4); wherein the receiving element for the syringe (5) is configured in the form of a tube (12) located on the side wall (13) of the hollow case (4) and is configured so that the first end of the tube (12) has an orifice for the syringe barrel (14), the second end of the tube (12) has an end wall (15) with an inlet channel (16) being intended for connecting a tip of the syringe barrel to the evaporation chamber (2) and the passage of the volatile anesthetic from the syringe barrel to the evaporation space of the hollow case (4); the connecting branch (6) is configured in the form of a pipe having a rounded or oval shape in the cross-section; wherein the outlet end with an outlet orifice (28) of the exhalation valve (9) is connected to the inlet end of the exhalation channel (10), an outlet end (31) of the exhalation channel (10) is connected to the inlet (33) of the filter (3); wherein the connecting branch (6), inhalation valve (8), and exhalation valve (9) are mutually spatially arranged so that the inhalation valve (8) and the exhalation valve (9) are positioned next to each other at a short distance from each other; and wherein the mixture of the volatile anesthetic vapor and air exits the outlet orifice (24) of the inhalation valve (8) and passes to the interior space of the connecting branch (6); and the gas exhaled by a human from the interior space of the connecting branch (6) enters the inlet (27) of the exhalation valve (9).

In one aspect, the inhalation device (1) in the inhalation system can comprise a mouthpiece to be mounted on the connecting branch (6) or inserted into the connecting branch (6).

In one aspect, a portion of the side wall (13) of the hollow case (4), on which the receiving element for the syringe (5) is positioned, can be configured in the form of a platform with a flat surface (36) being intended for the contacting with a lateral side of a syringe barrel flange with a flat surface.

In one aspect, the side wall (13) of the hollow case (4), on which the receiving element for the syringe (5) is positioned, can comprise a transverse groove with a rounded surface (37) being intended for inserting the lateral side of the syringe barrel flange with the rounded surface in it.

In one aspect, the inhalation device (1) in the inhalation system can comprise a button (41) being connected to the side wall (13) of the hollow case (4) and being able to move relatively to the side wall (13) of the hollow case (4), and being configured to drive a syringe piston; wherein the button (41) is configured to comprise at least one longitudinal connecting ledge (42) and at least one connecting groove (43) on the lateral side, and wherein the side wall (13) of the hollow case (4) comprises at least one longitudinal connecting groove (44) and at least one longitudinal connecting ledge (45); wherein the button (41) is connected to the side wall (13) of the hollow case (4) using the longitudinal connecting ledge (42), the connecting groove (43), the longitudinal connecting groove (44) and the longitudinal connecting ledge (45) so that the longitudinal connecting ledge (42) is spatially positioned in the longitudinal connecting groove (44), and the longitudinal connecting ledge (45) is spatially positioned in the connecting groove (43).

In one aspect, the button (41) can comprise at least one locking element (46), wherein each locking element (46) comprises a contact ledge (47); wherein a tube (12) of the receiving element for the syringe (5) comprises at least one series of the locking ledges (48) on the outer surface of the tube (12) of the receiving element for the syringe (5); wherein the locking element (46) and the series of the locking ledges (48) are configured to enable the contact ledge (47) to contact with the locking ledges (48), when the button (41) moves relatively to the side wall (13) of the hollow case (4).

In one aspect, the contact ledge (47) and the locking ledges (48) can be configured to enable the move of the button (41) relatively to the side wall (13) of the hollow case (4) in one direction only.

In one aspect, the syringe filled with the volatile anesthetic can comprise a syringe barrel, a piston positioned inside the syringe barrel, and a rod connected to the piston.

In one aspect, the syringe filled with the volatile anesthetic can comprise the syringe barrel and the piston positioned inside the syringe barrel, wherein the button (41) must comprise the rod (49) configured to drive the piston.

In one aspect, the rod (49) can have a cross-shaped cross-section.

In one aspect, the evaporation chamber (2) and the filter (3) can have a detachable connection with each other.

In one aspect, the inhalation system can comprise a sealed packaging comprising the inhalation device (1) and the syringe filled with the volatile anesthetic.

In one aspect, the inhalation system can comprise a first individual packaging comprising the inhalation device (1), comprise a second individual packaging comprising the syringe filled with the volatile anesthetic, and comprise a sealed packaging that comprise the first individual packaging comprising the inhalation device (1), and the second individual packaging comprising the syringe filled with the volatile anesthetic.

In one aspect, the syringe filled with the volatile anesthetic can comprise a protective cap mounted on the tip of the syringe barrel.

In one aspect, the syringe filled with the volatile anesthetic can be installed into the receiving element for the syringe (5).

In one aspect, the volatile anesthetic is methoxyflurane.

### BRIEF DESCRIPTION OF THE DRAWINGS

Hereinafter, the possible examples of the embodiments of the present disclosure are shown below using drawings in Figures 1-22 to better understand the essence of the present disclosure.
Fig. 1 shows a general view of the inhalation device.
Fig. 2 shows a front view of the inhalation device.
Fig. 3 shows a left view of the inhalation device.
Fig. 4 shows a rear view of the inhalation device.
Fig. 5 shows a top view of the inhalation device.
Fig. 6 shows an underside view of the inhalation device.
Fig. 7 shows a section A-A of Figure 4 without the syringe.
Fig. 8 shows a section A-A of Figure 4 with the syringe.
Fig. 9 shows a section D-D of Figure 3 without the syringe.
Fig. 10 shows a section D-D of Figure 3 with the syringe.
Fig. 11 shows a general view of the evaporation chamber (2) without the connecting branch (6), without the end wall (17) and without the filter (3).
Fig. 12 shows a left view of the evaporation chamber (2) without the connecting branch (6), without the end wall (17) and without the filter (3).
Fig. 13 shows a top view of the evaporation chamber (2) without the connecting branch (6), without the end wall (17) and without the filter (3).
Fig. 14 shows a view of the front portion of the evaporation chamber (2) with a 5 ml syringe installed.
Fig. 15 shows a view of the front portion of the evaporation chamber (2) with a 2 ml syringe installed.
Fig. 16 shows a section A-A of Figure 13.
Fig. 17 shows a section B-B of Figure 13.
Fig. 18 shows a general view of the button (41) with the rod (49).
Fig. 19 shows a front view of the button (41) with the rod (49).
Fig. 20 shows a right view of the button (41) with the rod (49).
Fig. 21 shows an underside view of the button (41) with the rod (49).
Fig. 22 shows a sectional view of the receiving element for the syringe (5) with the syringe installed and filled with the volatile anesthetic.

### DETAILED DESCRIPTION OF THE INVENTION

Herein, the terms "proximal" and "distal" are used.

The term "proximal" is used in the document to define an end of any element of the inhalation device (1) that, during the inhalation, is closer to the person performing the inhalation, and accordingly, the term "distal" is used to define an end of any element of the inhalation device (1), that, during the inhalation, is further from the person performing the inhalation.

In addition, the term "proximal" is used in the document to define an end of the syringe element, that, during the injection, is usually closer to the person performing the injection, and, accordingly, the term "distal" is used to define an end of the syringe element, that, during the injection, is further from the person performing the injection.

Herein, the term "syringe" means, unless otherwise specified, a medical syringe that is commonly used to perform injections. A state-of-the-art medical syringe consists of the basic elements, such as a syringe barrel, a piston positioned inside the syringe barrel, and a rod connected to the piston. The syringe barrel of the state-of-the-art medical syringe is usually configured to:
- have a cylindrical shape;
- comprise a flange at the proximal end of the barrel;
- comprise a tip at the distal end of the barrel intended for connecting the injection needle with the syringe barrel and for the passage of the injection liquid from the barrel to the interior channel of the injection needle.

The flange can be configured in two forms, the first form being a disc, and the second form being a truncated disc with two lateral sides with a rounded surface and two parallel lateral sides with a flat surface.

The piston of the state-of-the-art medical syringe is positioned inside the syringe barrel and configured to have a cylindrical shape. The lateral side of the piston comprises sealing elements that enables the piston to fit tightly to the interior wall of the syringe barrel.

The rod is configured to transmit the movement from a human finger to the piston and to drive the piston. The distal end of the rod is connected to the piston. The proximal end of the rod comprises a finger rest configured to absorb the force from a human finger.

The syringe to be used to perform the inhalation using the inhalation device according to the first disclosure has a specific feature, such as being filled with the volatile anesthetic.

It is necessary to note that:
- in the vast majority of the embodiments of the first disclosure, the inhalation device is configured for use of the above-mentioned syringe filled with the volatile anesthetic;
- in the vast majority of the embodiments of the second disclosure, the inhalation system comprises the above-mentioned syringe filled with the volatile anesthetic.

However, according to one of the embodiments of the second disclosure, the inhalation system comprises the syringe filled with the volatile anesthetic and configured to have the syringe barrel and the piston positioned inside the syringe barrel, wherein an element of the injection device (1), such as the button (41), must be configured to comprise the rod (49) intended to drive the piston. Accordingly, according to one of the embodiments of the first disclosure, the inhalation device is configured to enable using, inside the inhalation device, the syringe filled with the volatile anesthetic and configured to comprise the syringe barrel and the piston positioned in the syringe barrel, and to achieve this, the button (41) must be configured to comprise the rod (49) intended to drive the piston.

The first disclosure is the inhalation device (1).

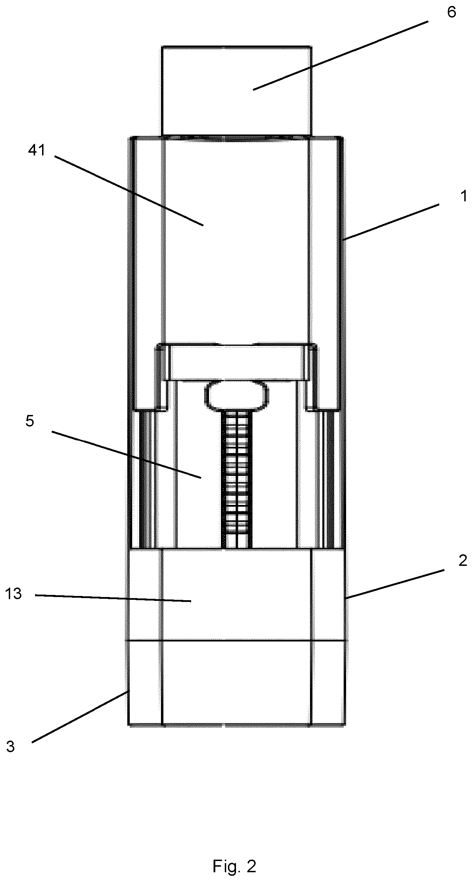

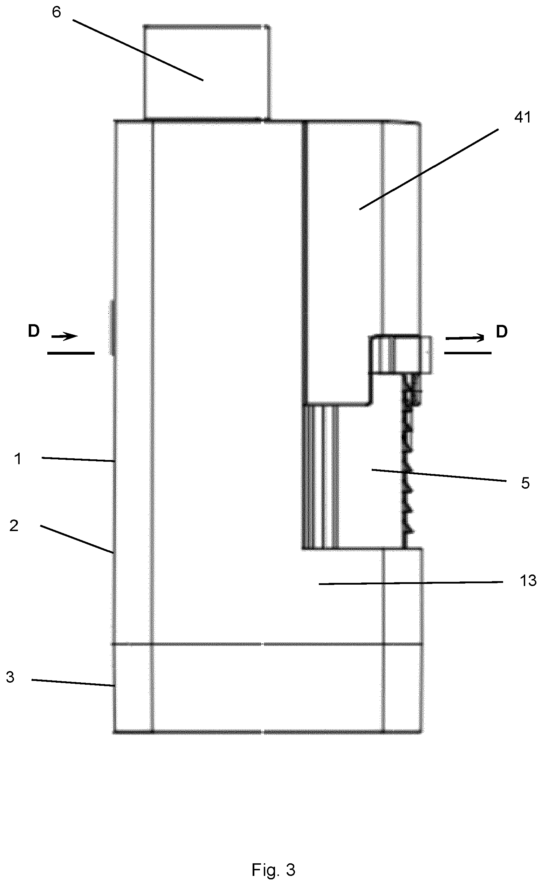

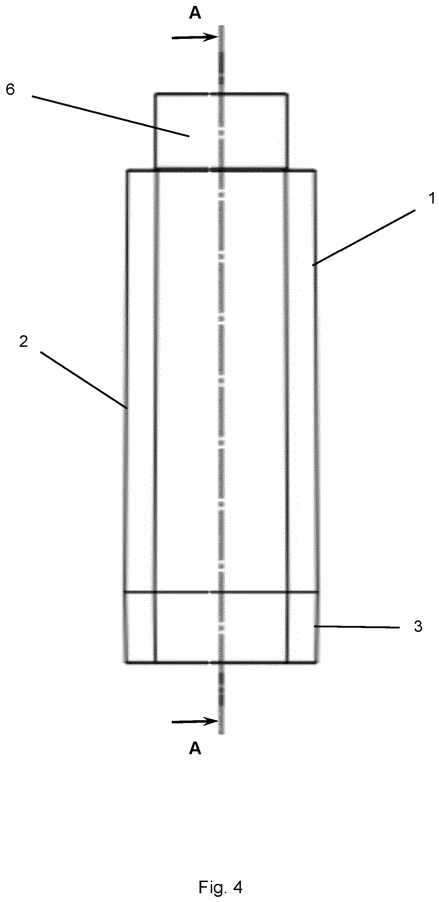

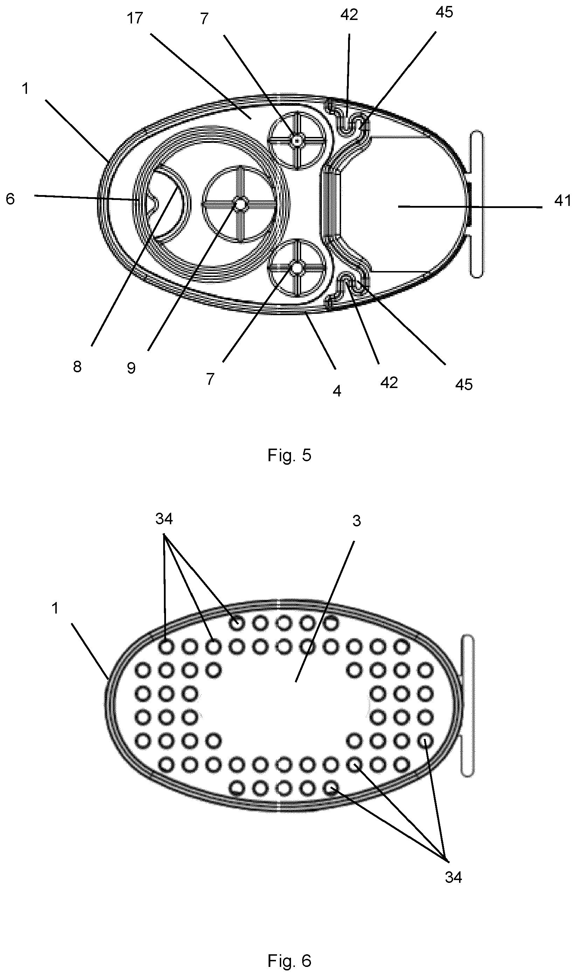

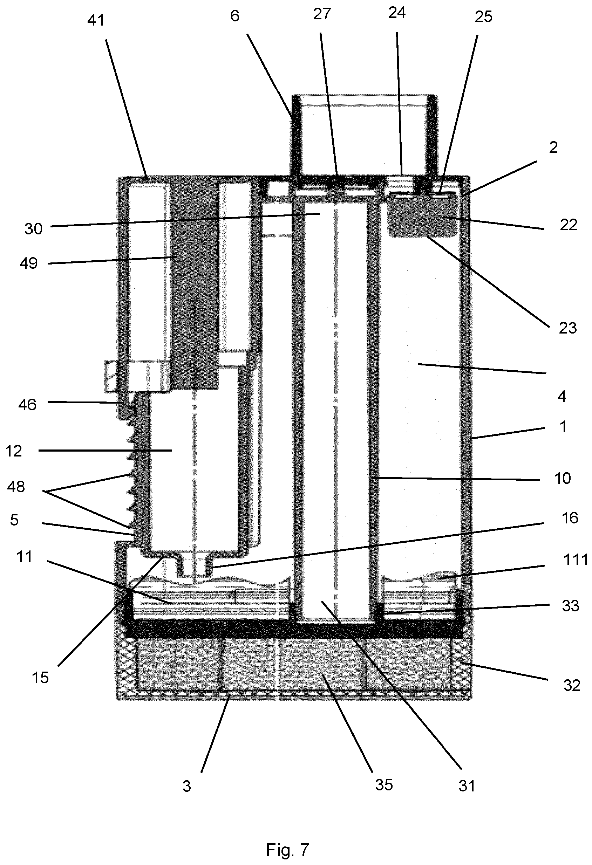

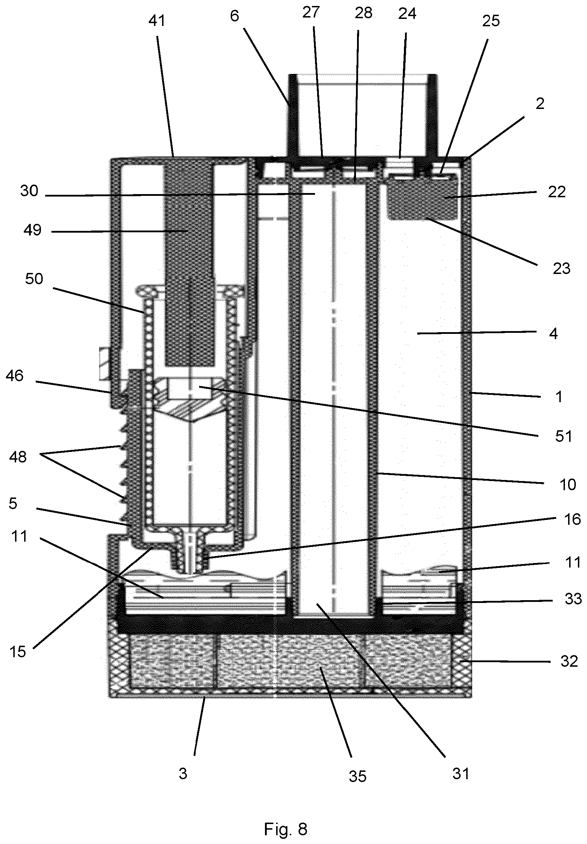

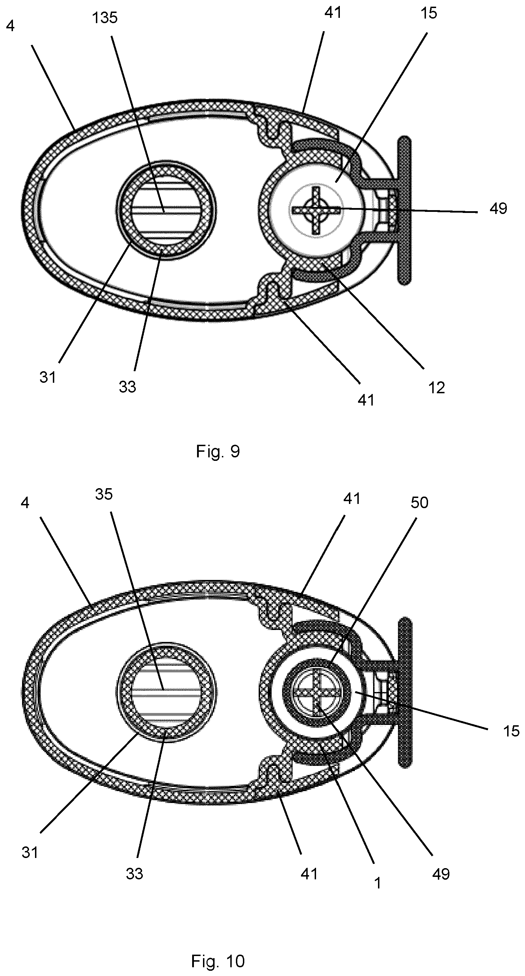

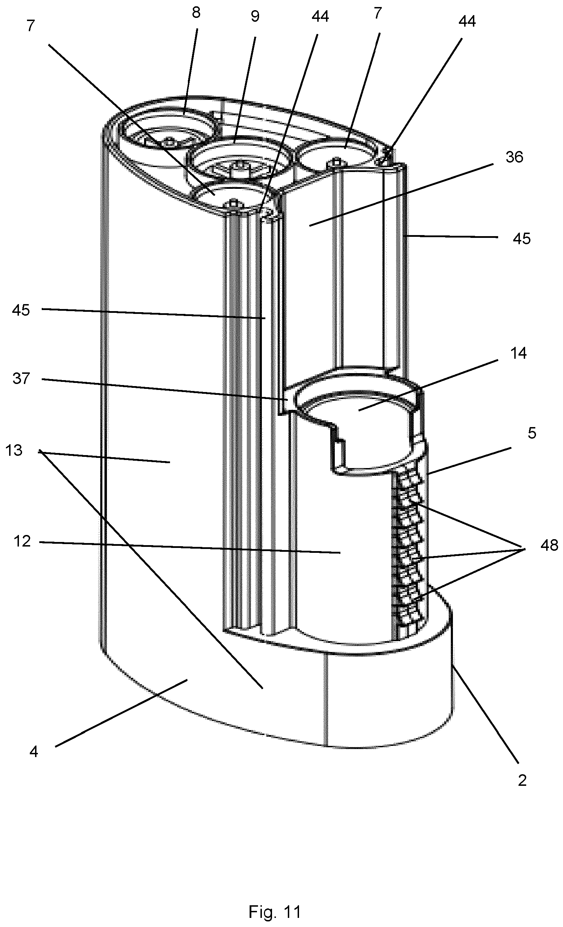

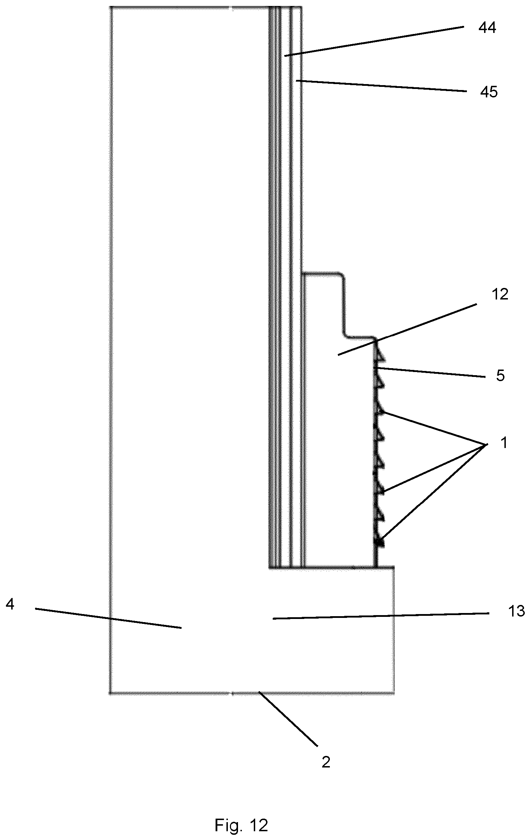

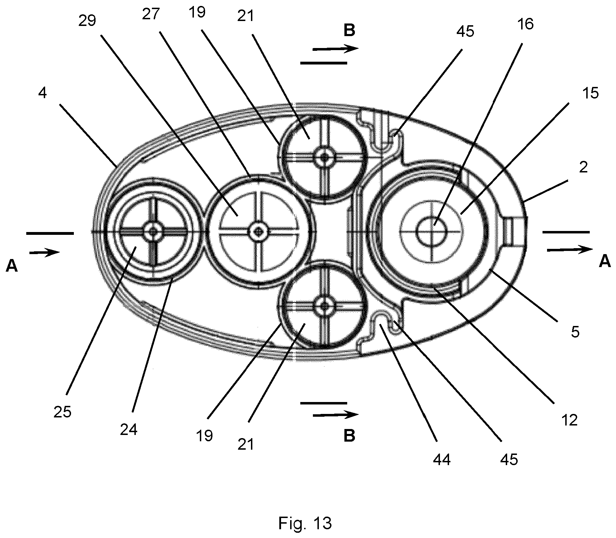

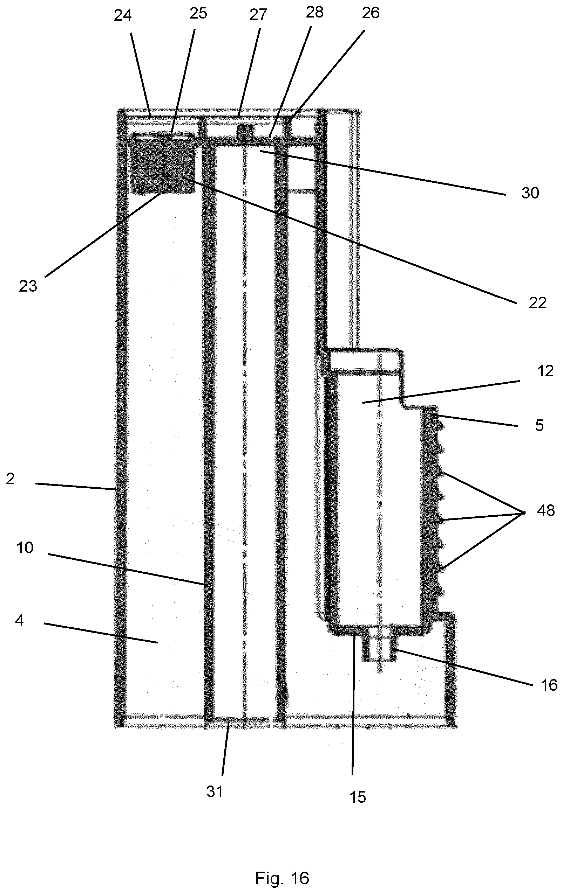

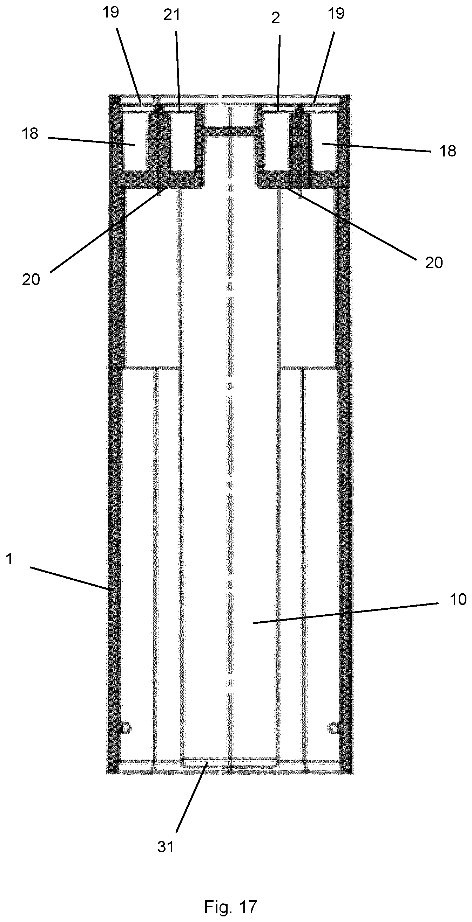

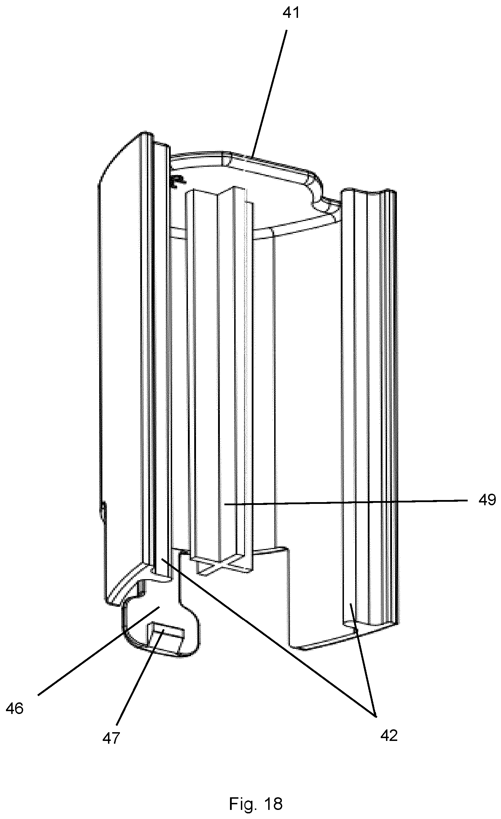

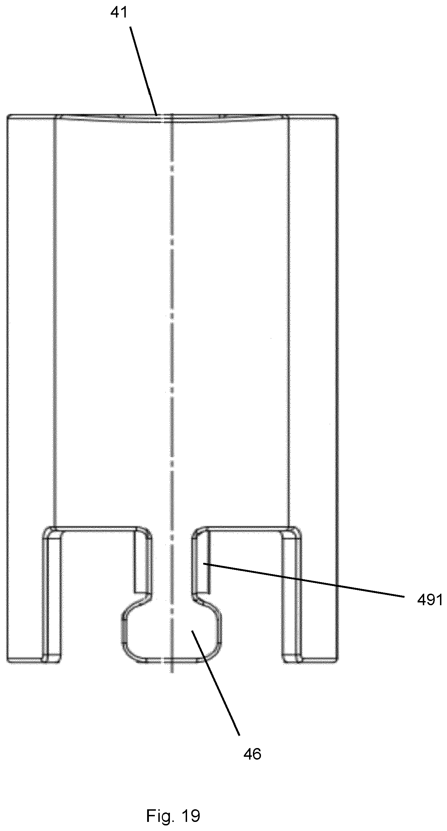

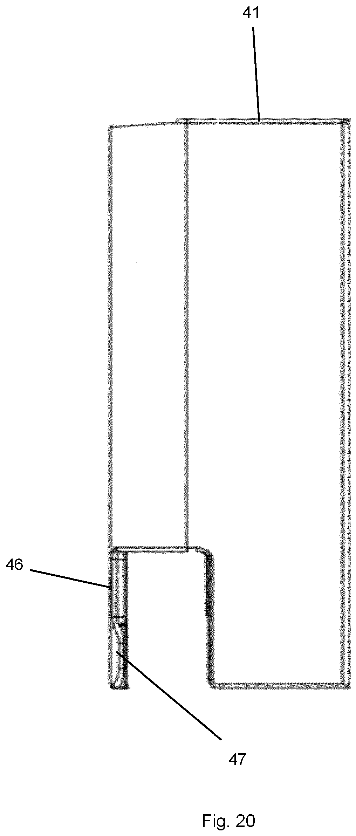

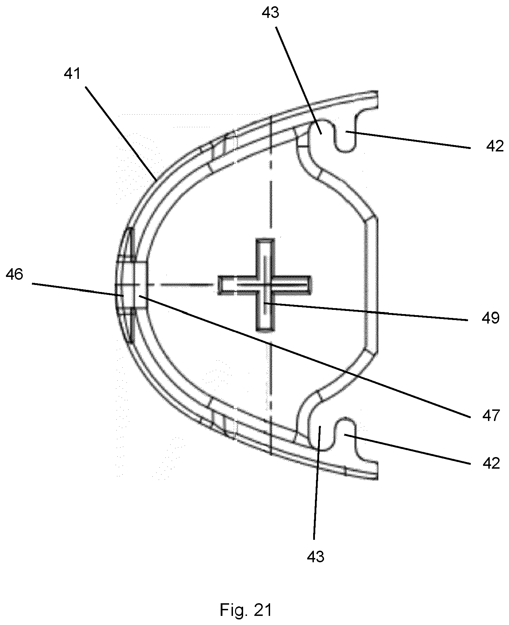

The embodiments of the inhalation device (1) according to the first disclosure are shown in Figures 1-22.

The inhalation device (1) according to the preferred embodiment of the first disclosure comprises the evaporation chamber (2) and the filter (3) connected to each other.

The evaporation chamber (2) is intended for vaporization of the volatile anesthetic, producing the mixture of the volatile anesthetic vapor and air, and feeding the mixture of the volatile anesthetic vapor and air to a human mouth. The evaporation chamber (2) has a complex structure, therefore, to better understand the structure of the evaporation chamber (2), the evaporation chamber (2) is shown in Figures without the connecting branch (6), without the end wall (17) and without the filter (3). The evaporation chamber (2) comprises the hollow case (4), the receiving element for the syringe (5) and the connecting branch (6). Two air valves (7), the inhalation valve (8), the exhalation valve (9), the exhalation channel (10), and the filler (11) are positioned inside the hollow case (4).

The hollow case (4) has walls that isolate the interior space from the environment. The exhalation channel (10) positioned in the hollow case (4) divides the interior space of the hollow case (4) into two spaces isolated from each other, the first space being the evaporation space, in which the evaporation of the volatile anesthetic and the formation of the mixture of the volatile anesthetic vapor and air occur, and the second space being the space in the exhalation channel (10) that is intended for the flow of the gas exhaled by a human to the filter (3).

The receiving element for the syringe (5) is configured to be installed with the syringe barrel filled with the volatile anesthetic and to feed the volatile anesthetic from the syringe to the evaporation space of the hollow case (4). The receiving element for the syringe (5) is configured in the form of a tube (12) positioned on the side wall (13) of the hollow case (4). The first end of the tube (12) has an orifice for the syringe barrel (14), and the second end of the tube (12) has an end wall (15) with the inlet channel (16). The end wall (15) is simultaneously the wall of the hollow case (4). The inlet channel (16) is intended to connect the tip of the syringe barrel to the evaporation chamber (2) and enable the flow of the volatile anesthetic from the syringe barrel to the evaporation space of the hollow case (4).

The connecting branch (6) is connected to the wall of the hollow case (4). The function of the connecting branch (6) is to feed the mixture of the volatile anesthetic vapor and air from the evaporation space of the hollow case (4) into a human mouth, and to remove the gas exhaled by a human from the human mouth into the space of the exhalation channel (10). The drawings show the embodiment of the connecting branch (6) position on the end wall (17) of the hollow case (4). For one skilled in the art, it is apparent that a different position of the connecting branch (6) at a different place on the wall of the hollow case (4) is possible. The connecting branch (6) is configured in the form of a tube, that can have a rounded or oval cross-section.

The air valves (7) are configured to feed a portion of the air from the environment into the evaporation space of the hollow case (4) when a human inhales. Each of the air valves (7) comprises a case (18) with an inlet end with an inlet orifice (19) and an outlet end with an outlet orifice (20), and a membrane (21) positioned inside the case (18). The inlet ends of the air valves (7) are connected to the walls of the hollow case (4) so that the inlet orifice (19) of each air valve (7) is an orifice in the wall of the hollow case (4), wherein the outlet end with the outlet orifice (20) of each air valve (7) are positioned inside the evaporation space of the hollow case (4). The drawings show an embodiment of the device with two air valves (7), and the arrangement of the air valves (7) that enable the inlet orifice (19) of each air valve (7) to be an orifice on the end wall (17) of the hollow case (4). It is apparent to one skilled in the art that it is possible to design the device with a different number of the air valves (7), such as with one air valve or with three air valves. It is also apparent to one skilled in the art that one or more air valves (7) can be positioned elsewhere on the wall of the hollow case (4).

The inhalation valve (8) is intended to feed a portion of the mixture of the volatile anesthetic vapor and air from the evaporation space of the hollow case (4) to the interior space of the connecting branch (6), when a human inhales. The inhalation valve (8) comprises a case (22) with an inlet end with an inlet orifice (23) and an outlet end with an outlet orifice (24), and a membrane (25) positioned inside the case (22). The outlet end of the inhalation valve (8) is connected to the wall of the hollow case (4) so that the outlet orifice (24) of the inhalation valve (8) is an orifice in the wall of the hollow case (4), and the inlet end is connected to the inlet orifice (23) of the inhalation valve (8) located in the evaporation space of the hollow case (4).

The exhalation valve (9) is intended to remove a portion of the gas exhaled by a human, when a human exhales, from the interior space of the connecting branch (6) into the space of the exhalation channel (10). The exhalation valve (9) comprises a case (26) with an inlet end with an inlet orifice (27) and an outlet end with an outlet orifice (28), and a membrane (29) positioned in the case (26). The inlet end of the exhalation valve (9) is connected to the wall of the hollow case (4) so that the inlet orifice (27) of the exhalation valve (9) is an orifice in the wall of the hollow case (4). The outlet end with the outlet orifice (28) of the exhalation valve (9) is connected to the inlet end of the exhalation channel (10).

The connecting branch (6), the inhalation valve (8) and the exhalation valve (9) are mutually spatially arranged such that the inhalation valve (8) and the exhalation valve (9) are positioned next to each other at a short distance from each other, and at the same time, the mixture of the volatile anesthetic vapor and air flows from the outlet orifice (24) of the inhalation valve (8) to the interior space of the connecting branch (6), and the gas exhaled by a human flows from the interior space of the connecting branch (6) to the inlet orifice (27) of the exhalation valve (9).

The exhalation channel (10) is configured in the form of a pipe comprising an inlet end (30) and an outlet end (31) and intended for the flow of the gas exhaled by a human to the filter (3). The exhalation channel (10) is positioned inside the hollow case so that the inlet end (30) of the exhalation channel (10) is connected to the outlet end with the outlet orifice (28) of the exhalation valve (9), wherein the outlet end (31) of the exhalation channel (10) is connected to the inlet of the filter (3).

The filter (3) is intended to clean the gas exhaled by a human from the volatile anesthetic vapors, and comprises a case (32), inside which the filter material (35) is positioned and adsorbs the volatile anesthetic vapors. Activated carbon, for example, can be used as the filter material. The case (32) of the filter (3) is connected to the hollow case (4) of the evaporation chamber (2). The connection of the case (32) of the filter (3) and the hollow case (4) can be fixed or detachable. When the connection of the case (32) of the filter (3) and the hollow case (4) is fixed, the device according to the present disclosure can be used once. When the connection of the case (32) of the filter (3) and the hollow case (4) is detachable, the filter (3) can be replaced, which allows using the device according to the present disclosure multiple times. The case (32) of the filter (3) comprises an inlet (33) connected to the outlet end (31) of the exhalation channel (10), and one or more outlet orifices (34), through which air purified from the volatile anesthetic vapor exits into the environment.

The filler (11) is positioned in the evaporation space of the hollow case (4). The filler (11) is configured of a porous material, wherein a layer of the material has thickness from 0.5 to several millimeters, for example, the material being a fibrous polypropylene material or a fibrous polyester material; and the filler (11) is configured to accelerate the evaporation of the volatile anesthetic fed to the evaporation space of the hollow case (4). Several layers of the filler (11) can be used.

The second disclosure is the inhalation system.

The inhalation system according to the preferred embodiment of the second disclosure includes the device (1) according to the preferred embodiment of the first disclosure and the syringe filled with the volatile anesthetic. The device (1) according to the preferred embodiment of the first disclosure comprises the evaporation chamber (2) and the filter (3). The evaporation chamber (2) is intended for vaporization of the volatile anesthetic, producing the mixture of the volatile anesthetic vapor and air, and feeding the mixture of the volatile anesthetic vapor and air to the human mouth. The evaporation chamber (2) comprises the hollow case (4), the receiving element for the syringe (5) and the connecting branch (6). Two air valves (7), the inhalation valve (8), the exhalation valve (9), the exhalation channel (10), and the filler (11) are positioned inside the hollow case (4).

The hollow case (4) has walls that isolate the interior space from the environment. The exhalation channel (10) positioned inside the hollow case (4) divides the interior space of the hollow case (4) into two spaces isolated from each other, wherein the first space being the evaporation space, in which the evaporation of the volatile anesthetic and the formation of the mixture of the volatile anesthetic vapor and air occur, and the second space being the space in the exhalation channel (10) that is intended for the flow of the gas exhaled by a human to the filter (3).

The receiving element for the syringe (5) is configured to be installed with the syringe barrel filled with the volatile anesthetic and to feed the volatile anesthetic from the syringe to the evaporation space of the hollow case (4). The receiving element for the syringe (5) is configured in the form of a tube (12) positioned on the side wall (13) of the hollow case (4). The first end of the tube (12) has an orifice for the syringe barrel (14), and the second end of the tube (12) has an end wall (15) with the inlet channel (16). The end wall (15) is simultaneously the wall of the hollow case (4). The inlet channel (16) is intended to connect the tip of the syringe barrel to the evaporation chamber (2) and enable the volatile anesthetic to flow from the syringe barrel to the evaporation space of the hollow case (4).

The connecting branch (6) is connected to the wall of the hollow case (4). The function of the connecting branch (6) is to feed the mixture of the volatile anesthetic vapor and air from the evaporation space of the hollow case (4) into a human mouth, and to remove the gas exhaled by a human from the human mouth into the space of the exhalation channel (10). The drawings show the embodiment of the arrangement of the connecting branch (6) on the end wall (17) of the hollow case (4). For one skilled in the art, it is apparent that the connecting branch (6) can have a different position at a different place on the wall of the hollow case (4). The connecting branch (6) is configured in the form of a tube, that can have a rounded or oval cross-section.

The air valves (7) are intended to feed a portion of the air from the environment into the evaporation space of the hollow case (4), when a human inhales. Each of the air valves (7) comprises a case (18) with an inlet end with an inlet orifice (19) and an outlet end with an outlet orifice (20), and a membrane (21) positioned inside the case (18). The inlet ends of the air valves (7) are connected to the walls of the hollow case (4) so that the inlet orifice (19) of each air valve (7) is an orifice in the wall of the hollow case (4), wherein the outlet end with the outlet orifice (20) of each air valve (7) are positioned inside the evaporation space of the hollow case (4). The drawings show an embodiment of the device with two air valves (7), and the arrangement of the air valves (7) that enable the inlet orifice (19) of each air valve (7) to be an orifice on the end wall (17) of the hollow case (4). It is apparent to one skilled in the art that the device can be configured to have a different number of the air valves (7), such as one air valve or with three air valves. It is also apparent to one skilled in the art that one or more air valves (7) can be positioned elsewhere on the wall of the hollow case (4).

The inhalation valve (8) is intended to feed a portion of the mixture of the volatile anesthetic vapor and air from the evaporation space of the hollow case (4) to the interior space of the connecting branch (6), when a human inhales. The inhalation valve (8) comprises a case (22) with an inlet end with an inlet orifice (23) and an outlet end with an outlet orifice (24), and a membrane (25) positioned inside the case (22). The outlet end of the inhalation valve (8) is connected to the wall of the hollow case (4) so that the outlet orifice (24) of the inhalation valve (8) is an orifice in the wall of the hollow case (4), and the inlet end is connected to the inlet orifice (23) of the inhalation valve (8) positioned inside the evaporation space of the hollow case (4).

The exhalation valve (9) is intended to remove a portion of the gas exhaled by a human, when a human exhales, from the interior space of the connecting branch (6) into the space of the exhalation channel (10). The exhalation valve (9) comprises a case (26) with an inlet end with an inlet orifice (27) and an outlet end with an outlet orifice (28), and a membrane (29) positioned inside the case (26). The inlet end of the exhalation valve (9) is connected to the wall of the hollow case (4) so that the inlet orifice (27) of the exhalation valve (9) is an orifice in the wall of the hollow case (4). The outlet end with the outlet orifice (28) of the exhalation valve (9) is connected to the inlet end of the exhalation channel (10).

The connecting branch (6), the inhalation valve (8) and the exhalation valve (9) are mutually spatially arranged such that the inhalation valve (8) and the exhalation valve (9) are positioned next to each other at a short distance from each other, and at the same time, the mixture of the volatile anesthetic vapor and air flows from the outlet orifice (24) of the inhalation valve (8) to the interior space of the connecting branch (6), and the gas exhaled by a human flows from the interior space of the connecting branch (6) to the inlet orifice (27) of the exhalation valve (9).

The exhalation channel (10) is configured in the form of a pipe comprising an inlet end (30) and an outlet end (31) and is intended for the flow of the gas exhaled by a human to the filter (3). The exhalation channel (10) is positioned inside the hollow case so that the inlet end (30) of the exhalation channel (10) is connected to the outlet end with the outlet orifice (28) of the exhalation valve (9), wherein the outlet end (31) of the exhalation channel (10) is connected to the inlet of the filter (3).

The filter (3) is intended to clean the gas exhaled by a human from the volatile anesthetic vapors, and comprises a case (32), inside which the filter material (35) is positioned and adsorbs the volatile anesthetic vapors. Activated carbon, for example, can be used as the filter material (35). The case (32) of the filter (3) is connected to the hollow case (4) of the evaporation chamber (2). The connection of the case (32) of the filter (3) and the hollow case (4) can be fixed or detachable. When the connection of the case (32) of the filter (3) and the hollow case (4) is fixed, the inhalation device according to the first disclosure can be used once. When the connection of the case (32) of the filter (3) and the hollow case (4) is detachable, the filter (3) can be replaced, which allows using the device according to the present disclosure multiple times. The case (32) of the filter (3) comprises an inlet (33) connected to the outlet end (31) of the exhalation channel (10), and one or more outlet orifices (34), through which air purified from the volatile anesthetic vapor exits into the environment.

The filler (11) is positioned in the evaporation space of the hollow case (4). The filler (11) is configured of a porous material, wherein a layer of the material has thickness from 0.5 to several millimeters, for example, the material being a fibrous polypropylene material or a fibrous polyester material; and the filler (11) is configured to accelerate the evaporation of the volatile anesthetic fed into the evaporation space of the hollow case (4). Several layers of the filler (11) can be used.

Any conventional syringe that is used for injections in medicine and that comprises a syringe barrel, a piston positioned inside the syringe barrel, and a rod connected to the piston, can be used as the syringe filled with the volatile anesthetic in the inhalation system, for example, a syringe having a volume of 2 ml or 5 ml. Methoxyflurane, that is a substance being a member of the class of the halogen-containing anesthetics, is preferably used as the volatile anesthetic.

An example of the use of the inhalation device (1) according to the preferred embodiment of the first disclosure and the use of the inhalation system according to the preferred embodiment of the second disclosure, when performing the inhalation, is described in Example 1.

### Example 1

A human takes the syringe filled with the volatile anesthetic, such as methoxyflurane, and inserts it in the receiving element for the syringe (5) so that the syringe barrel fits into the syringe barrel orifice (14) and the tip of the syringe barrel fits into the inlet channel (16).

After inserting the syringe filled with methoxyflurane into the inhalation device (1), a human takes the inhalation device (1) in the hand, and presses the finger rest on the rod, wherein a portion of methoxyflurane enters the evaporation chamber (2) through the tip of the syringe and the inlet channel (16), such as a portion of methoxyflurane enters the filler (11) positioned in the evaporation space of the hollow case (4). Due to the branched surface area of the filler material (11), rapid evaporation of methoxyflurane and the formation of methoxyflurane vapor occurs, wherein the methoxyflurane vapor enters the volume of the evaporation space of the hollow case (4), where methoxyflurane vapor mixes with air, and a mixture of the methoxyflurane vapor and air is formed in the evaporation space of the hollow case (4).

After that, a human brings the device to the mouth so that the end of the connecting branch (6) with the open orifice is positioned between the human lips, and alternately inhales and exhales.

During the phase of the inhalation, that a human performs, the following occurs in the inhalation device (1).

When a human inhales, a rarefaction occurs in the interior space of the connecting branch (6) leading to the opening of the inhalation valve (8). The air valves (7) open simultaneously with the inhalation valve (8). It happens in the following way. Under the rarefaction in the interior space of the connecting branch (6), the membrane (25) of the inhalation valve (8) deflects and allows the mixture of the methoxyflurane vapor and air to flow from the evaporation space of the hollow case (4) through the case (22) of the inhalation valve (8) and the outlet orifice (24) of the inhalation valve (8) into the interior space of the connecting branch (6). Further, during the inhalation, the mixture of the methoxyflurane vapor and air flows from the interior space of the connecting branch (6) into the human mouth and then the human lungs. When the inhalation valve (8) is opened, rarefaction also occurs in the evaporation space of the hollow case (4); under this rarefaction, the membranes (21) of the air valves (7) deflect and allow a portion of the air to flow from the environment through the inlet orifices (19) of the air valves (7) and cases (18) of the air valves (7) into the evaporation space of the hollow case (4). The portion of the air that entered the evaporation space of the hollow case (4) during the inhalation, due to the movement of this air, quickly mixes with the methoxyflurane vapor to form a mixture of methoxyflurane vapor and air.

The exhalation valve (9) is closed during the inhalation phase.

During the phase of exhalation, performed by a human, the following occurs in the inhalation device (1). When a human exhales, pressure arises in the interior space of the connecting branch (6) leading to the opening of the inhalation valve (9). It happens in the following way. Under the pressure in the interior space of the connecting branch (6), the membrane (29) of the inhalation valve (9) deflects and allows the gas exhaled by a human to flow from the interior space of the connecting branch (6) through the inlet orifice (27) of the inhalation valve (9) and the case (26) of the inhalation valve (9) into the space of the exhalation channel (10). When the exhalation valve (9) opens, pressure also arises in the space of the exhalation channel (10); under this pressure, the gas exhaled by a human, flows from the space of the exhalation channel (10) through the inlet (33) of the filter (3) into the case (32) of the filter (3), where it passes through a layer of the filter material (35). The gas exhaled by a human during the exhalation is essentially the air with a small amount of the methoxyflurane vapor. The filter material (35) cleans the air from methoxyflurane vapor, and the methoxyflurane vapor is absorbed by the filter material (35). The purified air exits through the outlet orifices (34) in the case (32) of the filter (3) to the environment.

The air valves (7) and the inhalation valve (8) are closed during the phase of the exhalation.

During the inhalation process, a human periodically, with specific time periods, presses the rod, injects a portion of methoxyflurane into the evaporation chamber (2), and inhales and exhales.

According to one of the embodiments of the second disclosure, the inhalation system can comprise the syringe filled with the volatile anesthetic that comprises protective cap mounted on the tip of the syringe barrel. The protective cap is intended to prevent leakage of the volatile anesthetic from the syringe during transportation and storage of the inhalation system until its use. In this embodiment of the injection kit, the injection kit is used for the inhalation similarly to Example 1 above, however, before inserting the syringe filled with the volatile anesthetic (methoxyflurane) into the inhalation device (1), a human removes the protective cap from the tip of the syringe barrel.

According to one of the embodiments of the present disclosure, the evaporation chamber (2) and the filter (3) in the inhalation device (1) can be configured to have detachable connection with each other. This arrangement of the said elements of the inhalation device (1) allows using the inhalation device (1) multiple times. The inhalation device (1) and the inhalation system according to this embodiment of the present disclosure are used for the inhalation similarly to Example 1, however, after the inhalation process is completed, a human further removes the used syringe from the inhalation device (1), then replaces the used filter to a filter that has not yet been used. To achieve this, a human detaches the filter used and the evaporation chamber from each other, and then attaches a filter that has not yet been used to the evaporation chamber. After that, the inhalation device (1) is ready for the next use for the inhalation. It is apparent and clear to one skilled in the art, that in this embodiment of the second disclosure, the inhalation system can comprise one more or more separate filters (3) not connected to the inhalation device (1) and comprise two or more syringes filled with the volatile anesthetic (methoxyflurane).

According to one of the embodiments of the present disclosure, the inhalation device (1) can comprise a mouthpiece mounted on the connecting branch (6) or inserted into the connecting branch (6). A mouthpiece of any known design can be used as the mouthpiece. Generally, mouthpieces are ergonomically shaped, as they are configured to fit into the human mouth and pass something into the human mouth. The presence of the mouthpiece in the inhalation device (1) makes the inhalation process more pleasant for a human. The inhalation device (1) according to this embodiment of the first disclosure and the inhalation system according to this embodiment of the second disclosure are used for the inhalation similarly to Example 1.

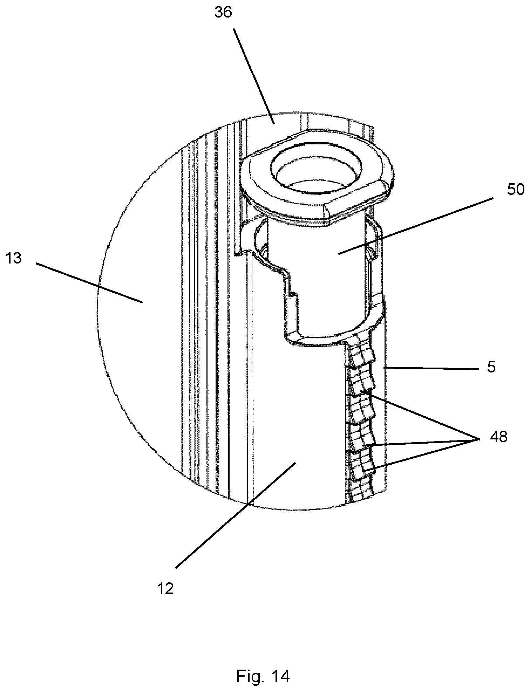

According to one of the embodiments of the present disclosure, in the inhalation device (1), a portion of the side wall (13) of the hollow case (4), on which the receiving element of the syringe (5) is positioned, can be configured in the form of the platform with the flat surface (36) intended to contact with the lateral side of the syringe barrel flange in the form of the cut disc. One of the issues of using the syringe filled with the volatile anesthetic is to securely fix the syringe barrel inside the receiving element of the syringe (5) and prevent the syringe barrel from popping out of the receiving element of the syringe (5) during the inhalation. This issue is caused by possible rotation of the syringe barrel during the inhalation process, which causes the tip of the syringe barrel to pop out of the inlet channel (16), to which the tip of the syringe barrel is connected. Some syringes, such as the 5 mL syringe (as shown in Figure 14), have a truncated disk-shaped flange of the syringe barrel that has two lateral sides with the rounded surface and two parallel lateral sides with the flat surface. The design of the side wall (13) of the hollow case (4) in the form of the platform with the flat surface (36) enables the syringe with the syringe barrel flange (50) in the form of the truncated disk with two lateral sides with the rounded surface and two parallel lateral sides with the flat surface to be fixed. When this syringe is being inserted into the receiving element for the syringe (5), one side of the syringe barrel flange (50) with the flat surface is pressed against the platform with the flat surface (36) on the side wall (13) of the hollow case (4), which prevents the syringe barrel installed into the receiving element for the syringe (5) from rotating.

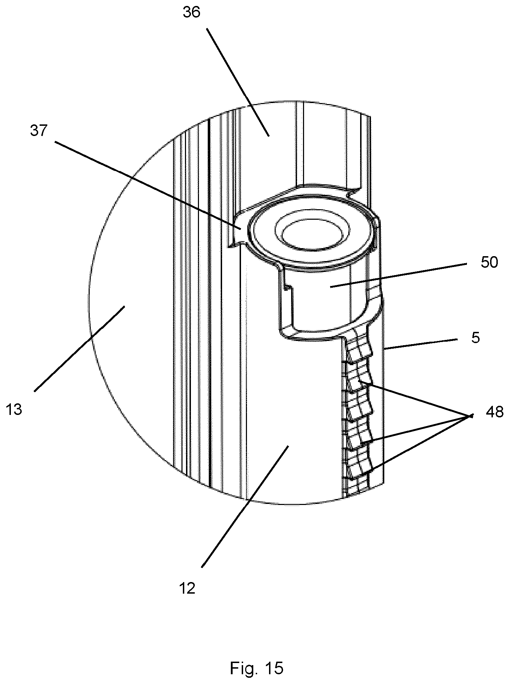

According to one of the embodiments of the present disclosure, the side wall (13) of the hollow case (4), on which the receiving element for the syringe (5) is positioned, of the inhalation device (1) can comprise a transverse groove with the rounded surface (37) configured to be inserted with the lateral side of the syringe barrel flange with the rounded surface. The transverse groove has a wall with a rounded surface and has side walls with a flat surface. Design of the transverse groove (37) on the side wall (13) of the hollow case (4) allows fixing the syringe that has the disk shape of the syringe barrel flange. This is the shape of the syringe barrel flange of the 2 ml syringe (as shown in Figure 15). When inserting this syringe into the receiving element for the syringe (5), the edge of the syringe barrel flange (50) enters the transverse groove with the rounded surface (37) so that the edge of the syringe barrel flange rests against the side wall of the transverse groove with the flat surface. The side wall of the transverse groove with the flat surface functions as a lock and prevents the syringe barrel from rising upward, which prevents the tip of the syringe barrel from popping out of the inlet channel (16) to which the tip of the syringe barrel is connected.

According to one of the embodiments of the present disclosure, the inhalation device (1) can comprise the button (41) connected to the side wall (13) of the hollow case (4), able to move relative to the side wall (13) of the hollow case (4) and configured to drive the piston of the syringe. One of the issues with using the syringe filled with the volatile anesthetic is that in small volume syringes, such as the 2 ml syringe, the finger rest positioned at the proximal end of the rod has a small area, resulting in the frequent release of the finger from the finger rest during the inhalation process. The button (41) has a much larger area compared to the area of the finger rest, which prevents the finger from popping off during the inhalation process. The button (41) is connected to the side wall (13) of the hollow case (4) and is able to move in such a way that the button (41) contacts the finger rest, and when the finger rests against the button (41), pressure is transferred to the finger rest. Accordingly, when the button (41) moves under the finger pressure, the rod connected to the piston moves, and the piston moves. To connect the button (41) with the side wall (13) of the hollow case (4), the lateral side of the button (41) and the side wall (13) of the hollow case (4) comprise the following elements:
the lateral side of the button (41) comprises two longitudinal connecting ledges (42) and two connecting grooves (43);
the side wall (13) of the hollow case (4) comprises two longitudinal connecting grooves (44) and two longitudinal connecting ledges (45).

It is clear to one skilled in the art that the number of the connecting elements, such as the longitudinal connecting ledge (42), the connecting groove (43), the longitudinal connecting groove (44) and the longitudinal connecting ledge (45), can be different. For example, there can be provided one longitudinal connecting ledge (42), one connecting groove (43), one longitudinal connecting groove (44) and one longitudinal connecting ledge (45).

The button (41) is connected to the side wall (13) of the hollow case (4) using the longitudinal connecting ledges (42), connecting grooves (43), longitudinal connecting grooves (44) and longitudinal connecting ledges (45) so that the longitudinal connecting ledge (42) is spatially positioned in the longitudinal connecting groove (44), and the longitudinal connecting ledge (45) is spatially positioned in the connecting groove (43). Longitudinal connecting ledges (42) and longitudinal connecting ledges (45) function as guides along which the button (41) moves.

The use of the inhalation device (1) according to said embodiment of the first disclosure and the inhalation system according to said embodiment of the second disclosure, when performing the inhalation, is described in Example 2.

### Example 2

A human lifts the button (41) mounted on the hollow case (4) so that there is enough space near the side wall (13) of the hollow case (4) to install the syringe filled with the volatile anesthetic, such as methoxyflurane. A human then takes the syringe filled with methoxyflurane and positions it in the receiving element for the syringe (5) so that the syringe barrel fits into the syringe barrel orifice (14) and the tip of the syringe barrel fits into the inlet channel (16).

After inserting the syringe filled with methoxyflurane into the inhalation device (1), a human takes the inhalation device (1) in the hand and presses the button (41), resulting in the movement of the rod and the movement of the piston connected to the rod, and, accordingly, a portion of methoxyflurane flows through the tip of the syringe and the inlet channel (16) into the evaporation chamber (2), specifically, a portion of methoxyflurane flows to the filler (11) positioned in the evaporation space of the hollow case (4). Due to the branched surface area of the filler material (11), rapid evaporation of methoxyflurane and formation of the methoxyflurane vapor occur, wherein the methoxyflurane vapor flows to the volume of the evaporation space of the hollow case (4), where it mixes with the air, and the mixture of the methoxyflurane vapor and air is formed in the evaporation space of the hollow case (4).

After that, a human brings the inhalation device (1) to the mouth so that the end of the connecting branch (6) with the open orifice is positioned between the human lips, and alternately inhales and exhales.

During the phase of the inhalation, that a human performs, the following occurs in the inhalation device (1).

When a human inhales, a rarefaction occurs in the interior space of the connecting branch (6) leading to the opening of the inhalation valve (8). The air valves (7) open simultaneously with the inhalation valve (8). It happens in the following way. Under the rarefaction in the interior space of the connecting branch (6), the membrane (25) of the inhalation valve (8) deflects and allows the mixture of the volatile anesthetic vapor and air to flow from the evaporation space of the hollow case (4) through the case (22) of the inhalation valve (8) and the outlet orifice (24) of the inhalation valve (8) into the interior space of the connecting branch (6). Further, during the inhalation, the mixture of the methoxyflurane vapor and air flows from the interior space of the connecting branch (6) into the human mouth and then the human lungs. When the inhalation valve (8) is opened, rarefaction also occurs in the evaporation space of the hollow case (4); under this rarefaction, the membranes (21) of the air valves (7) deflect and allow a portion of the air to flow from the environment through the inlet orifices (19) of the air valves (7) and cases (18) of the air valves (7) into the evaporation space of the hollow case (4). The portion of the air that entered the evaporation space of the hollow case (4) during the inhalation, due to the movement of this air, quickly mixes with the methoxyflurane vapor to form a mixture of methoxyflurane vapor and air.

The exhalation valve (9) is closed during the phase of the inhalation.

During the phase of the exhalation, performed by a human, the following occurs in the inhalation device (1). When a human exhales, pressure arises in the interior space of the connecting branch (6) leading to the opening of the inhalation valve (9). It happens in the following way. Under the pressure in the interior space of the connecting branch (6), the membrane (29) of the inhalation valve (9) deflects and allows the gas exhaled by a human to flow from the interior space of the connecting branch (6) through the inlet orifice (27) of the inhalation valve (9) and the case (26) of the inhalation valve (9) into the space of the exhalation channel (10). When the exhalation valve (9) opens, pressure also arises in the space of the exhalation channel (10); under this pressure, the gas exhaled by a human, flows from the space of the exhalation channel (10) through the inlet (33) of the filter (3) into the case (32) of the filter (3), where it passes through a layer of the filter material (35). The gas exhaled by a human during exhalation is essentially the air with a small amount of the methoxyflurane vapor. The filter material (35) cleans the air from methoxyflurane vapor, and the methoxyflurane vapor is absorbed by the filter material (35). The purified air exits through the outlet orifices (34) in the case (32) of the filter (3) to the environment.

The air valves (7) and the inhalation valve (8) are closed during the phase of the exhalation.

During the inhalation process, a human periodically, with specific time periods, presses the button (41), injects a portion of methoxyflurane into the evaporation chamber (2), and inhales and exhales.

In addition, according to one of the embodiments of the present disclosure, the button can be configured to comprise at least one locking element (46), wherein each locking element (46) comprises a contact ledge (47), wherein a tube (12) of the receiving element for the syringe (5) is configured to comprise at least one series of the locking ledges (48) on the outer surface of the tube (12) of the receiving element for the syringe (5). The Figures show one series of the locking ledges (48); however, there can be two series of the locking ledges (48) if the button (41) comprises two locking elements (46). The locking element (46) on the button (41) is configured in the form of a plate that can be bent. The said locking element (46) and the series of the locking ledges (48) are configured to enable the contact ledge (47) to contact the locking ledges (48), when the button (41) moves relative to the side wall (13) of the hollow case (4). One of the issues with using the syringe filled with the volatile anesthetic is that, during the inhalation, a human cannot see the syringe barrel comprising a volume scale. When pressing the finger against the rod or button (41), the movement of the rod, and, accordingly, the movement of the piston, is continuous. Therefore, a human does not control the volume of the portion of the volatile anesthetic every time they press the rod or button (41) and introduces the portions of the volatile anesthetic of different volumes into the evaporation chamber (2). The presence of the locking element (46) comprising the contact ledge (47) on the button (41) and the presence of the series of the locking ledges (48) on the outer surface of the tube (12) of the receiving element for the syringe (5) result in that when the button (41) moves relative to the side wall (13) of the hollow case (4), the contact ledge (47) periodically contacts with the locking ledges (48). When a human lightly presses the button (41), the button (41) first moves freely until the contact ledge (47) starts contacting with any locking ledge (48). When the contact ledge (47) contacts with the locking ledge (48), the button (41) retards, and the movement of the button (41) stops. At this point, if a human wants the button (41) to move further, a human must exert much more effort when pressing the button (41), and the following occurs:
first, the locking element (46) is bent, wherein when the button (41) is moving, the contact ledge (47) is moving along the surface of the locking ledge (48) until the moment when the contact ledge (47) losses contact with the locking ledge (48);
then the button (41) moves freely until the contact of the contact ledge (47) with the next locking ledge (48).

Such a cycle of the contacts of the contact ledge (47) with the locking ledges (48) during the movement of the button (41) results in the stepwise movement of the button (41) along the side wall (13) of the hollow case (4). When a human presses the button (41) with the finger during the inhalation process, they feel with the finger that the movement of the button (41) occurs in stepwise manner from one locking ledge (48) to the next locking ledge (48), which are arranged in a series. It is clear that the more steps of the button (41) a human feels, the larger the volume of the portion of the volatile anesthetic to be injected into the evaporation chamber (2) will be. By pressing the button (41) and simultaneously counting the number of the steps of the movement of the button (41), a human can both choose how many steps they want to move the button (41) and control the volume of the portion of the volatile anesthetic with each movement of the button (41).

The contact ledge (47) and the locking ledges (48) can be configured to enable the movement of the button (41) relative to the side wall (13) of the hollow case (4) in one direction only. This is possible, for example, when the contact ledge (47) is configured to have a triangle cross-section, in which one of the triangle sides is perpendicular to the surface of the locking element (46), and when each locking ledge (48) is configured to have a triangle cross-section, in which one of the triangle sides is perpendicular to the outer surface of the tube (12) of the receiving element for the syringe (5) (see Figure). This design of the contact ledge (47) and locking ledges (48) enables the movement of the button (41) in one direction only, which makes it impossible to reuse the inhalation device (1) after its first use for the inhalation process.

The inhalation device (1) and the inhalation system according to the said embodiment of the present disclosure, in which the movement of the button (41) is carried out in the stepwise manner, are used for inhalation similarly to Example 2.

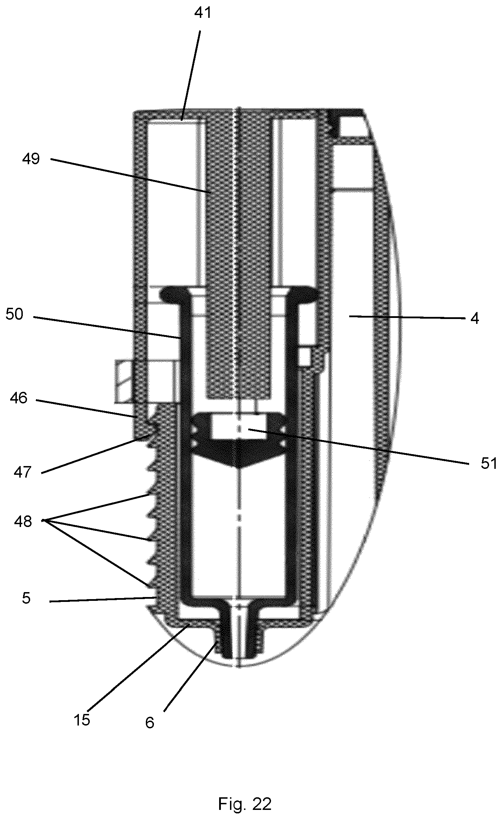

According to one of the embodiments of the second disclosure, the inhalation system comprises the syringe filled with the volatile anesthetic and configured to comprise the syringe barrel and the piston positioned inside the syringe barrel. If the inhalation system is used by a human who is not experienced with syringes, it is possible to accidentally press the rod, when inserting the syringe filled with the volatile anesthetic into the inhalation device (1), and the volatile anesthetic can leak out of the syringe and enter the air, which will be inhaled by bystanders. Such a situation is especially possible when a human experiences severe pain and severe stress resulting in poor control over their own movements. The use of the syringe comprising the syringe barrel and piston positioned inside the syringe barrel, and not comprising the rod connected to the piston, completely eliminates the possibility of uncontrolled leakage of the volatile anesthetic from the syringe as a result of the human actions, since a human is unable to drive the piston without an extra tool. In order to drive the piston, the button (41) must be configured to comprise the rod (49) configured to drive the piston. The rod (49) can be configured to have a cross-shaped cross-section. The view of the syringe filled with the volatile anesthetic according to this embodiment of the second disclosure is shown in Figure 22, where position (50) is the syringe barrel, and position (51) is the piston positioned in the syringe barrel (50). The inhalation device (1) and the inhalation system according to the said embodiment of the present disclosure, when using the syringe filled with the volatile anesthetic and configured to comprise the syringe barrel and the piston positioned in the syringe barrel, and the button (41) comprising the rod (49), is used for the inhalation similarly to Example 2. The dimensions of the hollow case (4) of the evaporation chamber (2) and the button (41) with the rod (49) are selected such that the syringe filled with the volatile anesthetic, and configured to comprise the syringe barrel and the piston positioned in the syringe barrel, could be inserted into the receiving element of the syringe (5) with the button (41) with the rod (49) in the upper position without removing the button (41) with the rod (49) from hollow case (4).

In order to reduce the number of Figures, Figures show the inhalation device (1) with the button (41) comprising the rod (49), and the button (41) comprising the rod (49). From the Figures provided, to one skilled in the art, the view and design of the button (41) without the rod (49) is clear.

The inhalation system can comprise packaging. Different embodiments of the inhalation system packaging are possible. According to one of the embodiments of the inhalation system packaging, the device (1) and the syringe filled with the volatile anesthetic can be packaged in a common sealed packaging, which can be sterile or non-sterile. According to another embodiment of the inhalation system packaging, the device (1) and the syringe filled with the volatile anesthetic can be packed in separate individual packagings, specifically, the inhalation system can comprise the sealed packaging comprising the first individual packaging, that comprises the inhalations device (1), and the second individual packaging, that comprises the syringe filled with the volatile anesthetic.

According to one of the embodiments of the second disclosure, the inhalation system can comprise the syringe filled with the volatile anesthetic and inserted into the receiving element for the syringe (5). In this embodiment of the second disclosure, the inhalation system is ready-to-use.

The technical contribution of the present disclosure resides in that that the inhalation device and the inhalation system prevent the volatile anesthetic vapor from entering the environment; enable the inhaler to adjust the doses of the volatile anesthetic fed to the inhalation device; expand the range of means for the inhalation with the volatile anesthetics, in particular, means for the inhalation with methoxyflurane.

The given examples only illustrate the present disclosure, but do not limit it.

## Claims

1. An inhalation device (1) comprising an evaporation chamber (2) and a filter (3) being connected to each other wherein the filter (3) is configured to comprise a case (32) with inlet (33) and at least one outlet (34), in which the filter material (35) being positioned; wherein the evaporation chamber (2) is configured to comprise a hollow case (4) having walls isolating the interior space from the environment, a receiving element for a syringe (5) is configured to be installed with a syringe barrel filled with a volatile anesthetic, a connecting branch (6) being connected to the wall of the hollow case (4), an exhalation channel (10) being located inside the hollow case (4) and dividing the interior space of the hollow case (4) into two spaces isolated from each other, such as the evaporation space, in which the evaporation of the volatile anesthetic and the formation of a mixture of the volatile anesthetic vapor and air occur, and the space in the exhalation channel (10) being intended for the flow of the gas exhaled by a human to the filter (3); at least one air valve (7) is configured to pass a portion of the air from the environment into the evaporation space of the hollow case (4) when a human inhales, and being positioned in the hollow case (4) so that the inlet end of the air valve (7) being connected to the walls of the hollow case (4) and the inlet orifice (19) of the air valve (7) being an orifice in the wall of the hollow case (4); an inhalation valve (8) is configured to pass a portion of the mixture of the volatile anesthetic vapor and air from the evaporation space of the hollow case (4) to the interior space of the connecting branch (6), when a human inhales, and being positioned in the hollow case (4) so that the outlet end of the inhalation valve (8) being connected to the wall of the hollow case (4), and the outlet orifice (24) of the inhalation valve (8) being an orifice in the wall of the hollow case (4); the exhalation valve (9) is configured to remove a portion of the gas exhaled by a human from the interior space of the connecting branch (6) into the space of the exhalation channel (10), when a human exhales, and being positioned in the hollow case (4) so that the inlet end of the exhalation valve (9) being connected to the wall of the hollow case (4), and the inlet orifice (27) of the exhalation valve (9) being an orifice in the wall of the hollow case (4); and a filler (11) being located in the evaporation space of the hollow case (4); wherein the receiving element for the syringe (5) is configured in the form of a tube (12) located on the side wall (13) of the hollow case (4) and is configured so that the first end of the tube (12) having an orifice for the syringe barrel (14), the second end of the tube (12) having an end wall (15) with an inlet channel (16) being intended for connecting a tip of the syringe barrel to the evaporation chamber (2) and the passage of the volatile anesthetic from the syringe barrel to the evaporation space of the hollow case (4); the connecting branch (6) is configured in the form of a pipe having a rounded or oval shape in the cross-section; wherein the outlet end with an outlet orifice (28) of the exhalation valve (9) being connected to the inlet end of the exhalation channel (10), an outlet end (31) of the exhalation channel (10) being connected to the inlet (33) of the filter (3); wherein the connecting branch (6), inhalation valve (8), and exhalation valve (9) being mutually spatially arranged so that the inhalation valve (8) and the exhalation valve (9) are positioned next to each other at a short distance from each other; and wherein the mixture of the volatile anesthetic vapor and air exiting the outlet orifice (24) of the inhalation valve (8) and passing to the interior space of the connecting branch (6); and the gas exhaled by a human from the interior space of the connecting branch (6) entering the inlet (27) of the exhalation valve (9).

2. The inhalation device (1) according to claim 1, ***characterised in that*** the inhalation device (1) further comprises a mouthpiece to be mounted on the connecting branch (6) or inserted into the connecting branch (6).

3. The inhalation device (1) according any of claims 1 to 2, ***characterised in that*** a portion of the side wall (13) of the hollow case (4), on which the receiving element for the syringe (5) is positioned, is configured in the form of a platform with a flat surface (36) being intended for the contacting with a lateral side of a syringe barrel flange with a flat surface.

4. The inhalation device (1) according any of claims 1 to 2, ***characterised in that*** the side wall (13) of the hollow case (4), on which the receiving element for the syringe (5) is positioned, comprises a transverse groove with a rounded surface (37) being intended for inserting the lateral side of the syringe barrel flange with the rounded surface in it.

5. The inhalation device (1) according any of claims 1 to 4, ***characterised in that*** the inhalation device (1) comprises a button (41) being connected to the side wall (13) of the hollow case (4) and being able to move relatively to the side wall (13) of the hollow case (4), and being configured to drive a syringe piston; wherein the button (41) is configured to comprise at least one longitudinal connecting ledge (42) and at least one connecting groove (43) on the lateral side, and wherein the side wall (13) of the hollow case (4) comprises at least one longitudinal connecting groove (44) and at least one longitudinal connecting ledge (45); wherein the button (41) is connected to the side wall (13) of the hollow case (4) using the longitudinal connecting ledge (42), the connecting groove (43), the longitudinal connecting groove (44) and the longitudinal connecting ledge (45) so that the longitudinal connecting ledge (42) is spatially positioned in the longitudinal connecting groove (44), and the longitudinal connecting ledge (45) is spatially positioned in the connecting groove (43).

6. The inhalation device (1) according to claim 5, ***characterised in that*** the button (41) comprise at least one locking element (46), wherein each locking element (46) comprises a contact ledge (47); wherein a tube (12) of the receiving element for the syringe (5) comprises at least one series of the locking ledges (48) on the outer surface of the tube (12) of the receiving element for the syringe (5); wherein the locking element (46) and the series of the locking ledges (48) are configured to enable the contact ledge (47) to contact with the locking ledges (48), when the button (41) moves relatively to the side wall (13) of the hollow case (4).

7. The inhalation device (1) according to claim 5, ***characterised in that*** the contact ledge (47) and the locking ledges (48) can be configured to enable the move of the button (41) relatively to the side wall (13) of the hollow case (4) in one direction only.

8. The inhalation device (1) according to any of claims 5 to 7, ***characterised in that*** the button (41) comprises a rod (49).

9. The inhalation device (1) according to claim 1, ***characterised in that*** volatile anesthetic is methoxyflurane.

10. An inhalation system comprising an inhalation device (1) according to any of claims 1 to 9 ***characterised in that*** it additionally comprises at least one syringe filled with the volatile anesthetic.

11. The inhalation system according to claim 10, ***characterised in that*** the inhalation device (1) comprises a mouthpiece to be mounted on the connecting branch (6) or inserted into the connecting branch (6).

12. The inhalation system according to any of claims 10 to 11, ***characterised in that*** a portion of the side wall (13) of the hollow case (4), on which the receiving element for the syringe (5) is positioned, can be configured in the form of a platform with a flat surface (36) being intended for the contacting with a lateral side of a syringe barrel flange with a flat surface.

13. The inhalation system according to any of claims 10 to 11, ***characterised in that*** the side wall (13) of the hollow case (4), on which the receiving element for the syringe (5) is positioned, can comprise a transverse groove with a rounded surface (37) being intended for inserting the lateral side of the syringe barrel flange with the rounded surface in it.

14. The inhalation system according to any of claims 10 to 13, ***characterised in that*** the inhalation device (1) comprises a button (41) being connected to the side wall (13) of the hollow case (4) and being able to move relatively to the side wall (13) of the hollow case (4), and being configured to drive a syringe piston; wherein the button (41) is configured to comprise at least one longitudinal connecting ledge (42) and at least one connecting groove (43) on the lateral side, and wherein the side wall (13) of the hollow case (4) comprises at least one longitudinal connecting groove (44) and at least one longitudinal connecting ledge (45); wherein the button (41) is connected to the side wall (13) of the hollow case (4) using the longitudinal connecting ledge (42), the connecting groove (43), the longitudinal connecting groove (44) and the longitudinal connecting ledge (45) so that the longitudinal connecting ledge (42) is spatially positioned in the longitudinal connecting groove (44), and the longitudinal connecting ledge (45) is spatially positioned in the connecting groove (43).

15. The inhalation system according to claim 14, ***characterised in that*** the button (41) comprises at least one locking element (46), wherein each locking element (46) comprises a contact ledge (47); wherein a tube (12) of the receiving element for the syringe (5) comprises at least one series of the locking ledges (48) on the outer surface of the tube (12) of the receiving element for the syringe (5); wherein the locking element (46) and the series of the locking ledges (48) are configured to enable the contact ledge (47) to contact with the locking ledges (48), when the button (41) moves relatively to the side wall (13) of the hollow case (4).

16. The inhalation system according to claim 15, ***characterised in that*** the contact ledge (47) and the locking ledges (48) can be configured to enable the move of the button (41) relatively to the side wall (13) of the hollow case (4) in one direction only.

17. The inhalation system according to any of claims 10 to 16, ***characterised in that*** the syringe filled with the volatile anesthetic can comprise a syringe barrel, a piston positioned inside the syringe barrel, and a rod connected to the piston.

18. The inhalation system according to any of claims 14 to 16, ***characterised in that*** the syringe filled with the volatile anesthetic can comprise a syringe barrel and a piston positioned inside the syringe barrel, wherein the button (41) must comprise the rod (49) configured to drive the piston.

19. The inhalation system according to any of the claims 10, 17 or 18 ***characterised in that*** the volatile anesthetic is methoxyflurane.

## Patentansprüche

1. Inhalationsvorrichtung (1) , die eine Verdampfungskammer (2) und einen Filter (3) umfasst, die miteinander verbunden sind, ***dadurch gekennzeichnet, dass*** der Filter (3) so konfiguriert ist, dass er ein Gehäuse (32) mit einem Einlass (33) und mindestens einem Auslass (34) umfasst, in dem das Filtermaterial (35) positioniert ist; wobei die Verdampfungskammer (2) so konfiguriert ist, dass sie ein hohles Gehäuse (4) mit Wänden umfasst, die den Innenraum von der Umgebung isolieren, ein Aufnahmeelement für die Spritze (5) so konfiguriert ist, dass es mit einem mit einem flüchtigen Anästhetikum gefüllten Spritzenzylinder installiert werden kann, ein Verbindungsstutzen (6) mit der Wand des hohlen Gehäuses (4) verbunden ist, ein Ausatmungskanal (10) sich innerhalb des hohlen Gehäuses befindet (4) angeordnet ist und den Innenraum des Hohlgehäuses (4) in zwei voneinander isolierte Räume unterteilt, nämlich den Verdampfungsraum, in dem die Verdampfung des flüchtigen Anästhetikums und die Bildung eines Gemisches aus dem flüchtigen Anästhesiedampf und Luft stattfindet, und den Raum im Ausatmungskanal (10), der für den Fluss des von einem Menschen ausgeatmeten Gases zum Filter (3) vorgesehen ist; mindestens ein Luftventil (7) so konfiguriert ist, dass es einen Teil der Luft aus der Umgebung in den Verdampfungsraum des Hohlgehäuses (4) leitet, wenn ein Mensch einatmet, und so im Hohlgehäuse (4) positioniert ist, dass das Einlassende des Luftventils (7) mit den Wänden des Hohlgehäuses (4) verbunden ist und die Einlassöffnung (19) des Luftventils (7) eine Öffnung in der Wand des Hohlgehäuses (4) ist; ein Inhalationsventil (8) so konfiguriert ist, dass es einen Teil des Gemisches aus flüchtigem Anästhesiedampf und Luft aus dem Verdampfungsraum des Hohlgehäuses (4) in den Innenraum des Verbindungsstücks (6) leitet, wenn ein Mensch einatmet, und so im Hohlgehäuse positioniert ist (4) so positioniert ist, dass das Auslassende des Inhalationsventils (8) mit der Wand des Hohlgehäuses (4) verbunden ist und die Auslassöffnung (24) des Inhalationsventils (8) eine Öffnung in der Wand des Hohlgehäuses (4) ist; das Ausatemventil (9) so konfiguriert ist, dass es einen Teil des von einem Menschen ausgeatmeten Gases aus dem Innenraum des Verbindungsstücks (6) in den Raum des Ausatmungskanals (10) entfernt, wenn ein Mensch ausatmet, und so im Hohlgehäuse (4) positioniert ist, dass das Einlassende des Ausatemventils (9) mit der Wand des Hohlgehäuses (4) verbunden ist, und die Einlassöffnung (27) des Ausatemventils (9) eine Öffnung in der Wand des Hohlgehäuses (4) ist; und ein Füllelement (11) im Verdampfungsraum des Hohlgehäuses (4) angeordnet ist; wobei das Aufnahmeelement für die Spritze (5) in Form eines an der Seitenwand (13) des Hohlgehäuses (4) angeordneten Rohrs (12) ausgebildet ist und so konfiguriert ist, dass das erste Ende des Rohrs (12) eine Öffnung für den Spritzenzylinder (14) aufweist, wobei das zweite Ende des Rohrs (12) eine Stirnwand (15) mit einem Einlasskanal (16) aufweist, der dazu bestimmt ist, eine Spitze des Spritzenzylinders mit der Verdampfungskammer (2) zu verbinden und den Durchgang des flüchtigen Anästhetikums vom Spritzenzylinder zum Verdampfungsraum des Hohlgehäuses (4) zu ermöglichen; der Verbindungsabzweig (6) in Form eines Rohrs mit einem runden oder ovalen Querschnitt ausgebildet ist; wobei das Auslassende mit einer Auslassöffnung (28) des Ausatemventils (9) mit dem Einlassende des Ausatmungskanals (10) verbunden ist, ein Auslassende (31) des Ausatmungskanals (10) mit dem Einlass (33) des Filters (3) verbunden ist; wobei der Verbindungsstutzen (6), das Einatemventil (8) und das Ausatemventil (9) räumlich so zueinander angeordnet sind, dass das Einatemventil (8) und das Ausatemventil (9) in geringem Abstand voneinander nebeneinander angeordnet sind; und wobei das Gemisch aus dem flüchtigen Anästhesiedampf und Luft aus der Auslassöffnung (24) des Einatemventils (8) austritt und in den Innenraum des Verbindungsstücks (6) gelangt; und das von einem Menschen aus dem Innenraum des Verbindungsstücks (6) ausgeatmete Gas in den Einlass (27) des Ausatemventils (9) eintritt.

2. Die Inhalationsvorrichtung (1) gemäß Anspruch 1, ***dadurch gekennzeichnet, dass*** die Inhalationsvorrichtung (1) ferner ein Mundstück umfasst, das an dem Verbindungsstutzen (6) angebracht oder in den Verbindungsstutzen (6) eingeführt werden kann.

3. Inhalationsvorrichtung (1) nach einem der Ansprüche 1 bis 2, ***dadurch gekennzeichnet, dass*** ein Abschnitt der Seitenwand (13) des Hohlgehäuses (4), an dem das Aufnahmeelement für die Spritze (5) positioniert ist, in Form einer Plattform mit einer flachen Oberfläche (36) ausgebildet ist, die zum Kontaktieren mit einer Seitenfläche eines Spritzenzylinderflansches mit einer flachen Oberfläche vorgesehen ist.

4. Inhalationsvorrichtung (1) nach einem der Ansprüche 1 bis 2, ***dadurch gekennzeichnet, dass*** die Seitenwand (13) des Hohlgehäuses (4), an der das Aufnahmeelement für die Spritze (5) angeordnet ist, eine Quernut mit einer abgerundeten Fläche (37) aufweist, die zum Einführen der Seitenfläche des Spritzenzylinderflansches mit der abgerundeten Fläche in diese bestimmt ist.

5. Inhalationsvorrichtung (1) nach einem der Ansprüche 1 bis 4, ***dadurch gekennzeichnet, dass*** die Inhalationsvorrichtung (1) einen Knopf (41) umfasst, der mit der Seitenwand (13) des Hohlgehäuses (4) verbunden ist und relativ zur Seitenwand (13) des Hohlgehäuses (4) beweglich ist und der zum Antreiben eines Spritzenkolbens konfiguriert ist; wobei der Knopf (41) so konfiguriert ist, dass er mindestens eine Längsverbindungsleiste (42) und mindestens eine Verbindungsnut (43) an der seitlichen Seite umfasst, und wobei die Seitenwand (13) des Hohlgehäuses (4) mindestens eine Längsverbindungsnut (44) und mindestens eine Längsverbindungsleiste (45) umfasst; wobei der Knopf (41) mit der Seitenwand (13) des Hohlgehäuses (4) unter Verwendung der Längsverbindungsleiste (42) verbunden ist, die Verbindungsnut (43), die Längsverbindungsnut (44) und die Längsverbindungsleiste (45) derart, dass die Längsverbindungsleiste (42) räumlich in der Längsverbindungsnut (44) positioniert ist und die Längsverbindungsleiste (45) räumlich in der Verbindungsnut (43) positioniert ist.

6. Die Inhalationsvorrichtung (1) gemäß Anspruch 5, ***dadurch gekennzeichnet, dass*** der Knopf (41) mindestens ein Verriegelungselement (46) umfasst, wobei jedes Verriegelungselement (46) eine Kontaktleiste (47) umfasst; wobei ein Rohr (12) des Aufnahmeelements für die Spritze (5) mindestens eine Reihe von Verriegelungsleisten (48) an der Außenfläche des Rohrs (12) des Aufnahmeelements für die Spritze (5) umfasst; wobei das Verriegelungselement (46) und die Reihe der Verriegelungsleisten (48) so konfiguriert sind, dass die Kontaktleiste (47) mit den Verriegelungsleisten (48) in Kontakt kommen kann, wenn sich der Knopf (41) relativ zur Seitenwand (13) des Hohlgehäuses (4) bewegt.

7. Die Inhalationsvorrichtung (1) gemäß Anspruch 5, ***dadurch gekennzeichnet, dass*** die Kontaktleiste (47) und die Verriegelungsleisten (48) so konfiguriert sein können, dass sie die Bewegung des Knopfes (41) relativ zur Seitenwand (13) des Hohlgehäuses (4) nur in einer Richtung ermöglichen.

8. Die Inhalationsvorrichtung (1) gemäß einem der Ansprüche 5 bis 7, ***dadurch gekennzeichnet, dass*** der Knopf (41) eine Stange (49) umfasst.

9. Das Inhalationsgerät (1) gemäß Anspruch 1, ***dadurch gekennzeichnet, dass*** das flüchtige Anästhetikum Methoxyfluran ist.

10. Inhalationssystem mit einer Inhalationsvorrichtung (1), ***dadurch gekennzeichnet, dass*** es zusätzlich mindestens eine mit dem flüchtigen Anästhetikum gefüllte Spritze umfasst; wobei eine Inhalationsvorrichtung (1) eine Verdampfungskammer (2) und einen Filter (3) umfasst, die miteinander verbunden sind; wobei der Filter (3) so konfiguriert ist, dass er ein Gehäuse (32) mit einem Einlass (33) und mindestens einem Auslass (34) umfasst, in dem sich das Filtermaterial (35) befindet; wobei die Verdampfungskammer (2) so konfiguriert ist, dass sie ein hohles Gehäuse (4) mit Wänden umfasst, die den Innenraum von der Umgebung isolieren, ein Aufnahmeelement für die Spritze (5) so konfiguriert ist, dass es mit einem mit einem flüchtigen Anästhetikum gefüllten Spritzenzylinder installiert werden kann, ein Verbindungsstutzen (6) mit der Wand des hohlen Gehäuses (4) verbunden ist, ein Ausatmungskanal (10) innerhalb des Hohlgehäuses (4) angeordnet ist und den Innenraum des Hohlgehäuses (4) in zwei voneinander isolierte Räume unterteilt, wie beispielsweise den Verdampfungsraum, in dem die Verdampfung des flüchtigen Anästhetikums und die Bildung eines Gemisches aus dem flüchtigen Anästhesiedampf und Luft stattfindet, und wobei der Raum im Ausatemkanal (10) für den Fluss des von einem Menschen ausgeatmeten Gases zum Filter (3) vorgesehen ist; mindestens ein Luftventil (7) ist so konfiguriert, dass es einen Teil der Luft aus der Umgebung in den Verdampfungsraum des Hohlgehäuses (4) leitet, wenn ein Mensch einatmet, und ist so im Hohlgehäuse (4) so positioniert ist, dass das Einlassende des Luftventils (7) mit den Wänden des Hohlgehäuses (4) verbunden ist und die Einlassöffnung (19) des Luftventils (7) eine Öffnung in der Wand des Hohlgehäuses (4) ist; ein Inhalationsventil (8), das so konfiguriert ist, dass es einen Teil des Gemisches aus flüchtigem Anästhesiedampf und Luft aus dem Verdampfungsraum des Hohlgehäuses (4) in den Innenraum des Verbindungsstücks (6) leitet, wenn ein Mensch einatmet, und das so im Hohlgehäuse (4) positioniert ist, dass das Auslassende des Inhalationsventils (8) mit der Wand des Hohlgehäuses (4) verbunden ist, und die Auslassöffnung (24) des Inhalationsventils (8) eine Öffnung in der Wand des Hohlgehäuses (4) ist; wobei das Ausatemventil (9) so konfiguriert ist, dass es einen Teil des von einem Menschen ausgeatmeten Gases aus dem Innenraum des Verbindungsstücks (6) in den Raum des Ausatmungskanals (10) entfernt, wenn ein Mensch ausatmet, und so im Hohlgehäuse (4) positioniert ist, dass das Einlassende des Ausatemventils (9) mit der Wand des Hohlgehäuses (4) verbunden ist, und die Einlassöffnung (27) des Ausatemventils (9) eine Öffnung in der Wand des Hohlgehäuses (4) ist; und wobei sich ein Füllelement (11) im Verdunstungsraum des Hohlgehäuses (4) befindet; wobei das Aufnahmeelement für die Spritze (5) in Form eines Rohrs (12) ausgebildet ist, das sich an der Seitenwand (13) des Hohlgehäuses (4) befindet, und so ausgebildet ist, dass das erste Ende des Rohrs (12) eine Öffnung für den Spritzenzylinder (14) aufweist, das zweite Ende des Rohrs (12) eine Stirnwand (15) mit einem Einlasskanal us (16) aufweist, der dazu bestimmt ist, eine Spitze des Spritzenzylinders mit der Verdampfungskammer (2) zu verbinden und den Durchgang des flüchtigen Anästhetikums vom Spritzenzylinder zum Verdampfungsraum des Hohlgehäuses (4) zu ermöglichen; der Verbindungszweig (6) in Form eines Rohrs mit einem runden oder ovalen Querschnitt ausgebildet ist; wobei das Auslassende mit einer Auslassöffnung (28) des Ausatemventils (9) mit dem Einlassende des Ausatmungskanals (10) verbunden ist, ein Auslassende (31) des Ausatmungskanals (10) mit dem Einlass (33) des Filters (3) verbunden ist; wobei der Verbindungsstutzen (6), das Einatemventil (8) und das Ausatemventil (9) räumlich so zueinander angeordnet sind, dass das Einatemventil (8) und das Ausatemventil (9) in geringem Abstand nebeneinander positioniert sind; und wobei das Gemisch aus dem flüchtigen Anästhesiedampf und Luft die Auslassöffnung (24) des Einatemventils (8) verlässt und in den Innenraum des Verbindungsstücks (6) gelangt; und das von einem Menschen aus dem Innenraum des Verbindungsstücks (6) ausgeatmete Gas in den Einlass (27) des Ausatemventils (9) gelangt.

11. Das Inhalationssystem gemäß Anspruch der 10, ***dadurch gekennzeichnet, dass*** die Inhalationsvorrichtung (1) ein Mundstück umfasst, das an dem Verbindungsstutzen (6) angebracht oder in den Verbindungsstutzen (6) eingeführt werden kann.

12. Das Inhalationssystem gemäß einem der Ansprüche 10 bis 11, ***dadurch gekennzeichnet, dass*** ein Teil der Seitenwand (13) des Hohlgehäuses (4), an dem das Aufnahmeelement für die Spritze (5) positioniert ist, in Form einer Plattform mit einer flachen Oberfläche (36) ausgebildet sein kann, die für den Kontakt mit einer seitlichen Seite eines Spritzenzylinderflansches mit einer flachen Oberfläche vorgesehen ist.

13. Das Inhalationssystem gemäß einem der Ansprüche 10 bis 11, ***dadurch gekennzeichnet, dass*** die Seitenwand (13) des Hohlgehäuses (4), an der das Aufnahmeelement für die Spritze (5) positioniert ist, eine Querrille mit einer abgerundeten Fläche (37) aufweisen kann, die zum Einführen der Seitenfläche des Spritzenzylinderflansches mit der abgerundeten Fläche in diese vorgesehen ist.

14. Das Inhalationssystem gemäß einem der Ansprüche 10 bis 13, ***dadurch gekennzeichnet, dass*** die Inhalationsvorrichtung (1) einen Knopf (41) umfasst, der mit der Seitenwand (13) des Hohlgehäuses (4) verbunden ist und sich relativ zur Seitenwand (13) des Hohlgehäuses (4) bewegen kann und der zum Antreiben eines Spritzenkolbens konfiguriert ist; wobei der Knopf (41) so konfiguriert ist, dass er mindestens eine Längsverbindungsleiste (42) und mindestens eine Verbindungsnut (43) an der seitlichen Seite umfasst, und wobei die Seitenwand (13) des Hohlgehäuses (4) mindestens eine Längsverbindungsnut (44) und mindestens eine Längsverbindungsleiste (45) umfasst; wobei der Knopf (41) mit der Seitenwand (13) des Hohlgehäuses (4) unter Verwendung der Längsverbindungsleiste (42) verbunden ist, die Verbindungsnut (43), die Längsverbindungsnut (44) und die Längsverbindungsleiste (45) derart, dass die Längsverbindungsleiste (42) räumlich in der Längsverbindungsnut (44) positioniert ist und die Längsverbindungsleiste (45) räumlich in der Verbindungsnut (43) positioniert ist.

15. Das Inhalationssystem nach Anspruch 14, ***dadurch gekennzeichnet, dass*** der Knopf (41) mindestens ein Verriegelungselement (46) umfasst, wobei jedes Verriegelungselement (46) eine Kontaktleiste (47) umfasst; wobei ein Rohr (12) des Aufnahmeelements für die Spritze (5) mindestens eine Reihe von Verriegelungsleisten (48) an der Außenfläche des Rohrs (12) des Aufnahmeelements für die Spritze (5) umfasst; wobei das Verriegelungselement (46) und die Reihe der Verriegelungsleisten (48) so konfiguriert sind, dass die Kontaktleiste (47) mit den Verriegelungsleisten (48) in Kontakt kommen kann, wenn sich der Knopf (41) relativ zur Seitenwand (13) des Hohlgehäuses (4) bewegt.

16. Das Inhalationssystem gemäß Anspruch 15, ***dadurch gekennzeichnet, dass*** die Kontaktleiste (47) und die Verriegelungsleisten (48) so konfiguriert sein können, dass sie die Bewegung des Knopfes (41) relativ zur Seitenwand (13) des Hohlgehäuses (4) nur in einer Richtung ermöglichen.

17. Das Inhalationssystem gemäß einem der Ansprüche 10 bis 16, ***dadurch gekennzeichnet, dass*** die mit dem flüchtigen Anästhetikum gefüllte Spritze einen Spritzenzylinder, einen im Spritzenzylinder angeordneten Kolben und eine mit dem Kolben verbundene Stange umfassen kann.

18. Das Inhalationssystem gemäß einem der Ansprüche 14 bis 16, ***dadurch gekennzeichnet, dass*** die mit dem flüchtigen Anästhetikum gefüllte Spritze den Spritzenzylinder und den innerhalb des Spritzenzylinders angeordneten Kolben umfassen kann, wobei der Knopf (41) die Stange (49) umfassen muss, die so konfiguriert ist, dass sie den Kolben antreibt.

19. Das Inhalationssystem gemäß einem der Ansprüche 10, 17 oder 18, ***dadurch gekennzeichnet, dass*** das flüchtige Anästhetikum Methoxyfluran ist.

## Revendications

1. Dispositif d'inhalation (1) comprenant une chambre d'évaporation (2) et un filtre (3) reliés l'un à l'autre, ***caractérisé en ce que*** le filtre (3) est configuré pour comprendre un boîtier (32) avec une entrée (33) et au moins une sortie (34), dans lequel le matériau filtrant (35) est positionné; la chambre d'évaporation (2) étant configurée pour comprendre un boîtier creux (4) ayant des parois isolant l'espace intérieur de l'environnement, un élément de réception pour la seringue (5) étant configuré pour être installé avec un cylindre de seringue rempli d'un anesthésique volatil, une branche de raccordement (6) étant reliée à la paroi du boîtier creux (4), un canal d'expiration (10) étant situé à l'intérieur du boîtier creux (4) et divisant l'espace intérieur du boîtier creux (4) en deux espaces isolés l'un de l'autre, tels que l'espace d'évaporation, dans lequel se produisent l'évaporation de l'anesthésique volatil et la formation d'un mélange de vapeur d'anesthésique volatil et d'air, et l'espace dans le canal d'expiration (10) destiné à l'écoulement du gaz expiré par un être humain vers le filtre (3); au moins une soupape d'air (7) est configurée pour laisser passer une partie de l'air de l'environnement dans l'espace d'évaporation du boîtier creux (4) lorsqu'un être humain inspire, et est positionnée dans le boîtier creux (4) de telle sorte que l'extrémité d'entrée de la soupape d'air (7) soit reliée aux parois du boîtier creux (4) et que l'orifice d'entrée (19) de la soupape d'air (7) étant un orifice dans la paroi du boîtier creux (4); une soupape d'inhalation (8) est configurée pour laisser passer une partie du mélange de vapeur d'anesthésique volatil et d'air provenant de l'espace d'évaporation du boîtier creux (4) vers l'espace intérieur de la branche de raccordement (6), lorsqu'un être humain inspire, et est positionnée dans le boîtier creux (4) de telle sorte que l'extrémité de sortie de la valve d'inhalation (8) soit reliée à la paroi du boîtier creux (4), et l'orifice de sortie (24) de la valve d'inhalation (8) étant un orifice dans la paroi du boîtier creux (4); la valve d'expiration (9) est configurée pour évacuer une partie du gaz expiré par un être humain de l'espace intérieur de la branche de raccordement (6) vers l'espace du canal d'expiration (10) lorsqu'un être humain expire, et est positionnée dans le boîtier creux (4) de telle sorte que l'extrémité d'entrée de la valve d'expiration (9) soit reliée à la paroi du boîtier creux (4), et l'orifice d'entrée (27) de la valve d'expiration (9) étant un orifice dans la paroi du boîtier creux (4); et un dispositif de remplissage (11) étant situé dans l'espace d'évaporation du boîtier creux (4); dans lequel l'élément de réception pour la seringue (5) est configuré sous la forme d'un tube (12) situé sur la paroi latérale (13) du boîtier creux (4) et est configuré de telle sorte que la première extrémité du tube (12) comporte un orifice pour le cylindre de la seringue (14), la deuxième extrémité du tube (12) comportant une paroi d'extrémité (15) avec un canal d'entrée (16) destiné à relier une pointe du cylindre de la seringue à la chambre d'évaporation (2) et au passage de l'anesthésique volatil du cylindre de la seringue à l'espace d'évaporation du boîtier creux (4); la branche de raccordement (6) est configurée sous la forme d'un tuyau ayant une section transversale arrondie ou ovale; dans lequel l'extrémité de sortie avec un orifice de sortie (28) de la valve d'expiration (9) est raccordée à l'extrémité d'entrée du canal d'expiration (10), une extrémité de sortie (31) du canal d'expiration (10) étant raccordée à l'entrée (33) du filtre (3); dans lequel la branche de raccordement (6), la valve d'inspiration (8) et la valve d'expiration (9) sont mutuellement agencées dans l'espace de telle sorte que la valve d'inspiration (8) et la valve d'expiration (9) sont positionnées l'une à côté de l'autre à une courte distance l'une de l' e l'autre; et dans lequel le mélange de vapeur d'anesthésique volatil et d'air sortant de l'orifice de sortie (24) de la valve d'inhalation (8) et passant dans l'espace intérieur de la branche de raccordement (6); et le gaz expiré par un être humain depuis l'espace intérieur de la branche de raccordement (6) entrant dans l'entrée (27) de la valve d'expiration (9).

2. Dispositif d'inhalation (1) selon la revendication 1, ***caractérisé en ce que*** le dispositif d'inhalation (1) comprend en outre un embout buccal à monter sur la branche de raccordement (6) ou à insérer dans la branche de raccordement (6).

3. Dispositif d'inhalation (1) selon l'une quelconque des revendications 1 à 2, ***caractérisé en ce* qu'**une partie de la paroi latérale (13) du boîtier creux (4), sur laquelle est positionné l'élément de réception pour la seringue (5), est configurée sous la forme d'une plate-forme avec une surface plane (36) destinée à entrer en contact avec un côté latéral d'une bride de cylindre de seringue avec une surface plane.

4. Dispositif d'inhalation (1) selon l'une quelconque des revendications 1 à 2, ***caractérisé en ce que*** la paroi latérale (13) du boîtier creux (4), sur laquelle est positionné l'élément de réception de la seringue (5), comprend une rainure transversale avec une surface arrondie (37) destinée à y insérer le côté latéral de la bride du cylindre de la seringue avec la surface arrondie.

5. Dispositif d'inhalation (1) selon l'une quelconque des revendications 1 à 4, ***caractérisé en ce que*** le dispositif d'inhalation (1) comprend un bouton (41) relié à la paroi latérale (13) du boîtier creux (4) et pouvant se déplacer par rapport à la paroi latérale (13) du boîtier creux (4), et étant configuré pour entraîner un piston de seringue; dans lequel le bouton (41) est configuré pour comprendre au moins un rebord de connexion longitudinal (42) et au moins une rainure de connexion (43) sur le côté latéral, et dans lequel la paroi latérale (13) du boîtier creux (4) comprend au moins une rainure de connexion longitudinale (44) et au moins un rebord de connexion longitudinal (45); dans lequel le bouton (41) est relié à la paroi latérale (13) du boîtier creux (4) à l'aide de la saillie de liaison longitudinale (42), la rainure de liaison (43), la rainure de liaison longitudinale (44) et le rebord de liaison longitudinal (45) de telle sorte que le rebord de liaison longitudinal (42) soit positionné spatialement dans la rainure de liaison longitudinale (44) et que le rebord de liaison longitudinal (45) soit positionné spatialement dans la rainure de liaison (43).

6. Dispositif d'inhalation (1) selon la revendication 5, ***caractérisé en ce que*** le bouton (41) comprend au moins un élément de verrouillage (46), chaque élément de verrouillage (46) comprenant un rebord de contact (47); dans lequel un tube (12) de l'élément de réception de la seringue (5) comprend au moins une série de rebords de verrouillage (48) sur la surface extérieure du tube (12) de l'élément de réception de la seringue (5); dans lequel l'élément de verrouillage (46) et la série de rebords de verrouillage (48) sont configurés pour permettre au rebord de contact (47) d'entrer en contact avec les rebords de verrouillage (48) lorsque le bouton (41) se déplace par rapport à la paroi latérale (13) du boîtier creux (4).

7. Dispositif d'inhalation (1) selon la revendication 5, ***caractérisé en ce que*** le rebord de contact (47) et les rebords de verrouillage (48) peuvent être configurés pour permettre le déplacement du bouton (41) par rapport à la paroi latérale (13) du boîtier creux (4) dans une seule direction.

8. Dispositif d'inhalation (1) selon l'une quelconque des revendications 5 à 7, ***caractérisé en ce que*** le bouton (41) comprend une tige (49).

9. Dispositif d'inhalation (1) selon la revendication 1, ***caractérisé en ce que*** l'anesthésique volatil est le méthoxyflurane.

10. Système d'inhalation comprenant un dispositif d'inhalation (1) ***caractérisé en ce* qu'**il comprend en outre au moins une seringue remplie de l'anesthésique volatil; dans lequel un dispositif d'inhalation (1) comprend une chambre d'évaporation (2) et un filtre (3) reliés l'un à l'autre; dans lequel le filtre (3) est configuré pour comprendre un boîtier (32) avec une entrée (33) et au moins une sortie (34), dans lequel se trouve le matériau filtrant (35); dans lequel la chambre d'évaporation (2) est configurée pour comprendre un boîtier creux (4) ayant des parois isolant l'espace intérieur de l'environnement, un élément de réception pour la seringue (5) est configuré pour être installé avec un cylindre de seringue rempli d'un anesthésique volatil, une branche de raccordement (6) étant reliée à la paroi du boîtier creux (4), un canal d'expiration (10) étant situé à l'intérieur du boîtier creux (4) et divisant l'espace intérieur du boîtier creux (4) en deux espaces isolés l'un de l'autre, tels que l'espace d'évaporation, dans lequel se produisent l'évaporation de l'anesthésique volatil et la formation d'un mélange de vapeur d'anesthésique volatil et d'air, et l'espace dans le canal d'expiration (10) étant destiné à l'écoulement du gaz expiré par un être humain vers le filtre (3); au moins une soupape d'air (7) est configurée pour laisser passer une partie de l'air de l'environnement dans l'espace d'évaporation du boîtier creux (4) lorsqu'un être humain inspire, et est positionnée dans le boîtier creux (4) de telle sorte que l'extrémité d'entrée de la soupape d'air (7) soit reliée aux parois du boîtier creux (4) et que l'orifice d'entrée (19) de la soupape d'air (7) soit un orifice dans la paroi du boîtier creux (4); une valve d'inhalation (8) étant configurée pour laisser passer une partie du mélange de vapeur d'anesthésique volatil et d'air provenant de l'espace d'évaporation du boîtier creux (4) vers l'espace intérieur de la branche de raccordement (6) lorsqu'un être humain inspire, et étant positionnée dans le boîtier creux (4) de telle sorte que l'extrémité de sortie de la valve d'inhalation (8) soit reliée à la paroi du boîtier creux (4), et l'orifice de sortie (24) de la valve d'inhalation (8) est un orifice dans la paroi du boîtier creux (4); la valve d'expiration (9) étant configurée pour évacuer une partie du gaz expiré par un être humain de l'espace intérieur de la branche de raccordement (6) vers l'espace du canal d'expiration (10) lorsqu'un être humain expire, et étant positionnée dans le boîtier creux (4) de telle sorte que l'extrémité d'entrée de la valve d'expiration (9) soit reliée à la paroi du boîtier creux (4), et l'orifice d'entrée (27) de la valve d'expiration (9) est un orifice dans la paroi du boîtier creux (4); et un dispositif de remplissage (11) étant situé dans l'espace d'évaporation du boîtier creux (4); dans lequel l'élément de réception pour la seringue (5) est configuré sous la forme d'un tube (12) situé sur la paroi latérale (13) du boîtier creux (4) et est configuré de telle sorte que la première extrémité du tube (12) comporte un orifice pour le cylindre de la seringue (14), la deuxième extrémité du tube (12) comporte une paroi d'extrémité (15) avec une canal e d'entrée (16) destinée à relier une pointe du cylindre de la seringue à la chambre d'évaporation (2) et au passage de l'anesthésique volatil du cylindre de la seringue à l'espace d'évaporation du boîtier creux (4); la branche de raccordement (6) est configurée sous la forme d'un tuyau ayant une section transversale arrondie ou ovale; dans lequel l'extrémité de sortie avec un orifice de sortie (28) de la valve d'expiration (9) est raccordée à l'extrémité d'entrée du canal d'expiration (10), une extrémité de sortie (31) du canal d'expiration (10) est raccordée à l'entrée (33) du filtre (3); dans lequel la branche de raccordement (6), la valve d'inspiration (8) et la valve d'expiration (9) sont mutuellement agencées dans l'espace de telle sorte que la valve d'inspiration (8) et la valve d'expiration (9) sont positionnées l'une à côté de l'autre à une courte distance l'une de l'autre; et dans lequel le mélange de vapeur d'anesthésique volatil et d'air sort de l'orifice de sortie (24) de la valve d'inhalation (8) et passe dans l'espace intérieur de la branche de raccordement (6); et le gaz expiré par un être humain depuis l'espace intérieur de la branche de raccordement (6) entre dans l'entrée (27) de la valve d'expiration (9).

11. Système d'inhalation selon l' u la revendication 10, ***caractérisé en ce que*** le dispositif d'inhalation (1) comprend un embout buccal à monter sur la branche de raccordement (6) ou à insérer dans la branche de raccordement (6).

12. Système d'inhalation selon l'une quelconque des revendications 10 à 11, ***caractérisé en ce* qu'**une partie de la paroi latérale (13) du boîtier creux (4), sur laquelle est positionné l'élément de réception pour la seringue (5), peut être configurée sous la forme d'une plate-forme avec une surface plane (36) destinée à entrer en contact avec un côté latéral d'une bride de cylindre de seringue avec une surface plane.

13. Système d'inhalation selon l'une quelconque des revendications 10 à 11, ***caractérisé en ce que*** la paroi latérale (13) du boîtier creux (4), sur laquelle est positionné l'élément de réception de la seringue (5), peut comprendre une rainure transversale avec une surface arrondie (37) destinée à y insérer le côté latéral de la bride du cylindre de la seringue avec la surface arrondie.

14. Système d'inhalation selon l'une quelconque des revendications 10 à 13, ***caractérisé en ce que*** le dispositif d'inhalation (1) comprend un bouton (41) relié à la paroi latérale (13) du boîtier creux (4) et pouvant se déplacer par rapport à la paroi latérale (13) du boîtier creux (4), et étant configuré pour entraîner un piston de seringue; dans lequel le bouton (41) est configuré pour comprendre au moins un rebord de connexion longitudinal (42) et au moins une rainure de connexion (43) sur le côté latéral, et dans lequel la paroi latérale (13) du boîtier creux (4) comprend au moins une rainure de connexion longitudinale (44) et au moins un rebord de connexion longitudinal (45); dans lequel le bouton (41) est relié à la paroi latérale (13) du boîtier creux (4) à l'aide de la saillie de liaison longitudinale (42), la rainure de liaison (43), la rainure de liaison longitudinale (44) et le rebord de liaison longitudinal (45) de telle sorte que le rebord de liaison longitudinal (42) soit positionné spatialement dans la rainure de liaison longitudinale (44) et que le rebord de liaison longitudinal (45) soit positionné spatialement dans la rainure de liaison (43).

15. Système d'inhalation selon la revendication 14, ***caractérisé en ce que*** le bouton (41) comprend au moins un élément de verrouillage (46), chaque élément de verrouillage (46) comprenant un rebord de contact (47); dans lequel un tube (12) de l'élément de réception de la seringue (5) comprend au moins une série de rebords de verrouillage (48) sur la surface extérieure du tube (12) de l'élément de réception de la seringue (5); dans lequel l'élément de verrouillage (46) et la série de rebords de verrouillage (48) sont configurés pour permettre au rebord de contact (47) d'entrer en contact avec les rebords de verrouillage (48) lorsque le bouton (41) se déplace par rapport à la paroi latérale (13) du boîtier creux (4).

16. Système d'inhalation selon la revendication 15, ***caractérisé en ce que*** le rebord de contact (47) et les rebords de verrouillage (48) peuvent être configurés pour permettre le déplacement du bouton (41) par rapport à la paroi latérale (13) du boîtier creux (4) dans une seule direction.

17. Système d'inhalation selon l'une quelconque des revendications 10 à 16, ***caractérisé en ce que*** la seringue remplie d'anesthésique volatil peut comprendre un cylindre de seringue, un piston positionné à l'intérieur du cylindre de seringue et une tige reliée au piston.

18. Le système d'inhalation selon l'une quelconque des revendications 14 à 16, ***caractérisé en ce que**,* la seringue remplie d'anesthésique volatil peut comprendre le cylindre de seringue et le piston positionné à l'intérieur du cylindre de seringue, dans lequel le bouton (41) doit comprendre la tige (49) configurée pour entraîner le piston.

19. Le système d'inhalation selon l'une quelconque des revendications 10, 17 ou 18, ***caractérisé en ce que*** l'anesthésique volatil est le méthoxyflurane.
